# EUROPEAN PATENT APPLICATION

(11) **EP 3 939 421 A1**
(43) Date of publication of application: **19.01.2022**
(21) Application number: 20769214.6
(22) Date of filing: 10.03.2020
(51) Int. Cl.: A01K 43/00, G01N 21/27, G01N 21/85, G06T 7/00

(54) **EGG CLASSIFICATION DEVICE, EGG CLASSIFICATION METHOD, AND COMPUTER PROGRAM**

(30) Priority: 13.03.2019 JP 2019046262
(71) Applicant: Nabel Co., Ltd., Kyoto-shi, Kyoto 601-8444 (JP)
(72) Inventor: KASHIMORI, Ayuko, Kyoto-shi, Kyoto 601-8444 (JP); SAIDA, Kazuki, Kyoto-shi, Kyoto 601-8444 (JP); MOMII, Santa, Kyoto-shi, Kyoto 601-8444 (JP)
(74) Representative: Cabinet Beaumont
(86) International application number: PCT/JP2020/010191
(87) International publication number: WO 2020/184542

(57) **Abstract**

Provided are an egg sorting device that can sort eggs more suitably than a conventional device, an egg sorting method and a computer program.

The egg sorting device includes an image data acquisition unit that acquires image data representing an image obtained by photographing an egg; a first determination unit that determines a state of an egg according to image data acquired by the image data acquisition unit by a learning model trained using training data including information indicating a state of an egg and image data related to the egg; and a decision unit that decides a classification of the egg according to a determination result of a state of an egg.

## Description

### [Technical Field]

The present invention relates to an egg sorting device for sorting eggs, an egg soring method and a computer program.

### [Background Art]

Eggs with the eggshells are collected, washed, sorted depending on the size, packed in packages and then shipped. An egg with the eggshell is simply referred to as an egg below. Eggs are hen's eggs, for example. Eggs also include eggs with the eggshells other than hen's eggs. Eggs are checked for the presence or absence of dirt, the degree of dirt, the presence or absence of damage of the eggshell and the degree of damage. Moreover, eggs are checked for an interior abnormal egg such as an egg containing blood, an egg with broken yolk, a Yolk-less egg or a rotten egg or the like. Depending on the check results, eggs are sorted before shipment. For example, eggs having the damaged eggshells and eggs having the eggshells with dirt adhering even after washing are sorted to a classification, such as a classification for eggs to be shipped to a different destination, different from a classification for eggs with no damage and no dirt. Various devices have been developed for the purpose of checking eggs. Patent Document 1 discloses a technique for determining the presence or absence of dirt on the eggshells based on an image obtained by photographing eggs.

### [Prior Art Document]

### [Patent Document]

[Patent Document 1] Japanese Patent No. 4734620

### [Summary of Invention]

### [Problems to be Solved by Application]

There are eggs with the eggshells of various color such as white, brown or the like, not the same color. Some eggs have patterns or protrusions different from damage or dirt. When eggs are sorted, these various types of eggs are sometimes mixed up, which may make it difficult to accurately check and sort the eggs based on the images thereof.

The present invention is made in view of these circumstances, and the object is to provide an egg sorting device that can sort eggs more suitably than a conventional device, an egg sorting method and a computer program.

### [Means for Solving Problems]

An egg sorting device according to the present invention is characterized by comprising: an image data acquisition unit that acquires image data representing an image obtained by photographing an egg; a first determination unit that determines a state of the egg according to the image data acquired by the image data acquisition unit by a learning model trained using training data including information indicating a state of an egg and image data related to the egg; and a decision unit that decides a classification of an egg according to a determination result of a state of the egg.

In the egg sorting device according to the present invention, it is characterized in that the determination result indicates a degree of damage of an eggshell, a degree of dirt on an eggshell or a type of dirt on an eggshell.

In the egg sorting device according to the present invention, it is characterized in that the image data acquisition unit acquires image data representing an image obtained by photographing the egg contained in a package, and the determination result indicates a degree of damage of an eggshell, a degree of dirt on an eggshell or a type of dirt on an eggshell.

The egg sorting device according to the present invention is characterized by further comprising an output unit that outputs information indicating the state of the egg determined by the first determination unit.

The egg sorting device according to the present invention is characterized by further comprising a second determination unit that determines a state of the egg according to the image data acquired by the image data acquisition unit based on a predefined rule, wherein the decision unit decides a classification of the egg depending on a determination result obtained by the first determination unit and a determination result obtained by the second determination unit.

The egg sorting device according to the present invention is characterized by further comprising: an acceptance unit that accepts information indicating a state of an egg whose state is identified; and a retraining unit that retrains the learning model using training data including the image data acquired by the image data acquisition unit for the same egg and the information accepted by the acceptance unit.

The egg sorting device according to the present invention is characterized by further comprising: a first information acquisition unit that acquires information designating quality of an egg; and a first adjustment unit that selects a learning model to be used in the first determination unit out of multiple learning models according to the information acquired or that adjusts a criterion for the decision unit deciding a classification of an egg depending on the determination result according to the information acquired.

The egg sorting device according to the present invention is characterized by further comprising: a second information acquisition unit that acquires information indicating an estimated elapsed time from egg-laying; and a second adjustment unit that selects a learning model to be used by the first determination unit out of multiple learning models according to the information acquired or that adjusts a criterion for the decision unit deciding a classification of an egg depending on the determination result according to the information acquired.

An egg sorting method according to the present invention is characterized by comprising: acquiring image data representing an image obtained by photographing an egg; determining a state of the egg according to the image data acquired by a learning model trained using training data including information indicating a state of an egg and image data related to the egg; and deciding a classification of an egg according to a determination result of a state of the egg.

A computer program that causes a computer to execute processing of sorting eggs according to the present invention is characterized by causing the computer to execute a process including: a step of determining a state of an egg according to acquired image data of the egg by a learning model trained using training data including image data representing an image obtained by photographing an egg and information indicating a state of the egg; and a step of deciding a classification of an egg according to a determination result of a state of the egg.

In the present invention, the egg sorting device acquires image data representing an image obtained by photographing an egg, determines a state of the egg according to the image data by a learning model, and decides a classification of the egg depending on the determination result. By using the learning model for determining the state of the egg, it is possible to accurately determine the state of eggs and suitably sort the eggs.

In one embodiment of the present invention, the egg sorting device determines the degree of damage of the eggshell, the degree of dirt on the eggshell or a type of dirt on the eggshell as a state of an egg. The degree of damage of the eggshell, the degree of dirt on the eggshell or a type of dirt on the eggshell have much effect on the quality of the eggs. By sorting eggs depending on the state of the eggs, eggs with a suitable quality can be selected.

In one embodiment of the present invention, the egg sorting device determines the states of eggs such as the degree of damage of the eggshell, the degree of dirt on the eggshell or a type of dirt on the eggshell according to image data representing an image obtained by photographing the eggs contained in the package. By determining the states of the eggs even contained in the package and sorting the eggs, it is possible to prevent eggs of poor quality from being shipped.

In one embodiment of the present invention, the egg sorting device outputs the information indicating the states of the determined eggs. This makes it possible to inform the user of the states of the eggs. Furthermore, another device can use the determination result of the states of the eggs in order to sort the eggs.

In one embodiment of the present invention, a combination of the rule-based determination and the determination using the learning model enables accurate determination of eggs and suitable sorting of the eggs.

In one embodiment of the present invention, the egg sorting device retrains the learning model using the information indicating the identified state of an egg and the image data representing an image of an egg as training data. The learning model is retrained according to the actual state of eggs, and the learning model can be updated so as to more accurately determine the state of eggs.

In one embodiment of the present invention, the egg sorting device adjusts the learning model used for determining the state of the egg or adjusts the criteria for deciding a classification of eggs, according to the designated egg quality. Thus, eggs can be suitably sorted so as to acquire eggs with necessary quality.

In one embodiment of the present invention, the egg sorting device adjusts the learning model used for determining the state of the egg or adjusts the criteria for deciding a classification of an egg, according to a predicted elapsed time from egg-laying. This makes it possible to suitably sort eggs.

### [Effects of Invention]

The present invention produces a great effect such as more accurate determination of the states of eggs and more suitable sorting of the eggs even in the case where various eggs having different states coexist.

### [Brief Description of Drawings]

FIG. 1 is a schematic view illustrating an example of the configuration of an egg sorting system utilized for sorting and shipping eggs.
FIG. 2 is a block diagram illustrating an example of the functional configuration of a broken egg detection device.
FIG. 3 is a schematic view illustrating an example of the configuration of an image acquisition unit.
FIG. 4 is a flowchart showing a processing procedure for sorting eggs performed by an analysis unit of the broken egg detection device.
FIG. 5 is a flowchart showing a procedure for determination processing.
FIG. 6 is a conceptual diagram illustrating an example of the functional configuration of a learning model.
FIG. 7 is a block diagram illustrating an example of the functional configuration of an egg image analysis device.
FIG. 8 is a schematic view illustrating an example of the configuration of an image acquisition unit.
FIG. 9 is a flowchart showing a processing procedure for sorting eggs.
FIG. 10 is a flowchart showing a procedure for determination processing.
FIG. 11 is a conceptual diagram illustrating an example of the functional configuration of a learning model.
FIG. 12 is a flowchart showing a procedure for setting processing.
FIG. 13 is a block diagram illustrating an example of the functional configuration of an inside-package-image analysis device.
FIG. 14 is a schematic view illustrating an example of the configuration of an image acquisition unit.
FIG. 15 is a conceptual diagram illustrating an example of the functional configuration of a learning model.
FIG. 16 is a flowchart showing a processing procedure for retraining.
FIG. 17A is a schematic view illustrating a screen example to be displayed on a display unit upon retraining.
FIG. 17B is a schematic view illustrating a screen example to be displayed on the display unit upon retraining.
FIG. 17C is a schematic view illustrating a screen example to be displayed on the display unit upon retraining.
FIG. 17D is a schematic view illustrating a screen example to be displayed on the display unit upon retraining.
FIG. 18 is a schematic view illustrating another screen example to be displayed on the display unit upon retraining.
FIG. 19 is a block diagram illustrating an example of the functional configuration of the egg image analysis device which training is performed outside thereof.
FIG. 20 is a block diagram illustrating an example of the internal configuration of a storage device.

### [Mode for Carrying out Invention]

The present invention will be described below with reference to the drawings depicting embodiments thereof.

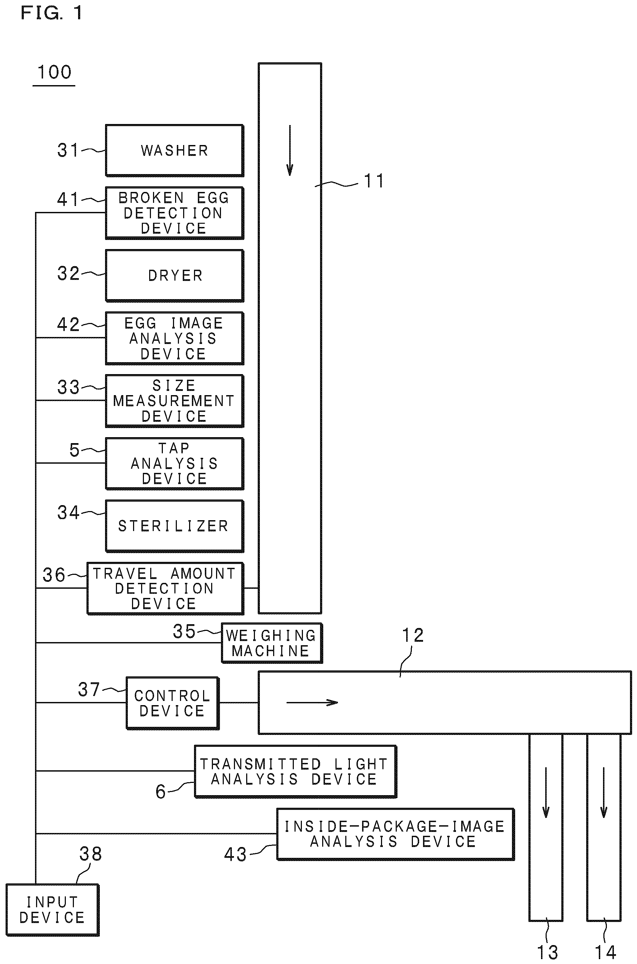

FIG. 1 is a schematic view illustrating an example of the configuration of an egg sorting system 100 utilized for sorting and shipping eggs. A large number of eggs are collected from a production site such as a poultry farm or the like and processed by the egg sorting system 100. The egg sorting system 100 includes a conveyance device 11 for linearly conveying eggs. The conveyance device 11 is composed of a conveyance path for placing a large number of eggs thereon and conveying the placed eggs. The conveyance device 11 is a conveyor with multiple rollers, for example, places eggs on the rollers and coveys the eggs by movement of the rollers. The conveyance direction of eggs is indicated by an arrow in FIG. 1. The large number of eggs collected are subject to various processing while being conveyed by the conveyance device 11.

Each of the eggs is placed on the conveyance path with its longitudinal axis direction intersecting the conveyance direction. Eggs are conveyed while rotating regarding the axis along the longitudinal axis direction as the axis of rotation. For example, the rollers on which eggs are placed are rotated to rotate the eggs. Multiple eggs are conveyed while aligned in a row along the conveyance direction. Moreover, eggs are conveyed while multiple rows of the eggs are aligned in the direction intersecting the conveyance direction.

The conveyance device 11 is connected to a travel amount detection device 36. The travel amount detection device 36 measures the motion of the conveyance device 11 and detects the travel amount of each of the eggs conveyed by the conveyance device 11 based on the motion of the conveyance device 11. For example, the travel amount detection device 36 is provided with a sensor for detecting passing of each of the rollers formed of the roller conveyor and detects the movement of each of rollers based on the detection results by the sensor. The travel amount detection device 36 detects the travel amount of each of the eggs that is being conveyed based on the detected movement of the rollers. Moreover, the travel amount detection device 36 outputs information indicating the travel amount of each of the eggs.

The egg sorting system 100 includes a washer 31. The washer 31 washes the eggs conveyed by the conveyance device 11. The egg sorting system 100 includes a broken egg detection device 41. The broken egg detection device 41 detects a broken egg included in the eggs conveyed after having been washed. The broken egg is an egg the eggshell of which is damaged. The broken egg detection device 41 shoots an image of each of the eggs and determines whether or not the egg is a broken egg by an image analysis. Furthermore, the broken egg detection device 41 removes the egg determined to be a broken egg from the conveyance device 11. Moreover, the broken egg detection device 41 may determine the degree of damage of the eggshell. The egg sorting system 100 includes a dryer 32. The dryer 32 dries the eggs conveyed by the conveyance device 11.

The egg sorting system 100 includes an egg image analysis device 42. The egg image analysis device 42 shoots an image of each of the eggs conveyed by the conveyance device 11 and determines the state of each of the eggs by an image analysis. For example, the egg image analysis device 42 determines, based on the image of an egg obtained by photographing an egg, the color of the eggshell, the presence or absence of dirt on the eggshell, the degree of dirt, the type of dirt or the surface properties. Furthermore, the egg image analysis device 42 may measure, based on the image of an egg, the shape or size of the egg. For example, the egg image analysis device 42 measures, from the image of an egg, lengths of a longitudinal axis and a lateral axis of the egg as values indicating the shape of the egg. For example, the egg image analysis device 42 measures, based on the image of an egg, an area of the egg as a value indicating the size of the egg. The egg image analysis device 42 outputs information indicating the state of each of the eggs.

The egg sorting system 100 includes a size measurement device 33. The size measurement device 33 measures the size of each of the eggs conveyed by the conveyance device 11. For example, the size measurement device 33 has a light emitting device and a light receiving device, measures a time during which light from the light emitting device is cut off by an egg and fails to be received by the light receiving device, and measures the length of the egg in the conveyance direction based on the measured time and the travel rate of the egg by the conveyance device 11.

The egg sorting system 100 includes a tap analysis device 5. The tap analysis device 5 taps each of the eggs conveyed by the conveyance device 11, measures a phenomenon caused by tapping and determines the state of the eggshell based on the measurement result. For example, the tap analysis device 5 has a learning model trained using training data including tapping data indicating the phenomenon caused by tapping an egg and information indicating the state of the eggshell. The tap analysis device 5 acquires tapping data indicating a phenomenon caused by tapping an egg, determines the state of the eggshell based on the acquired tapping data using the learning model and decides a classification of the egg based on the determination result. The tap analysis device 5 measures sound caused by tapping an egg. Furthermore, the tap analysis device 5 determines the presence or absence of damage on the eggshell, the size of damage, the thickness of the eggshell, the strength of the eggshell, the rigidity of the eggshell or the like based on the measurement result. The tap analysis device 5 outputs the information indicating the state of the eggshell.

The egg sorting system 100 includes a sterilizer 34 for sterilizing eggs conveyed by the conveyance device 11. For example, the sterilizer 34 irradiates eggs with ultraviolet rays to sterilize the surface of the eggshell. The washer 31, the broken egg detection device 41, the dryer 32, the egg image analysis device 42, a weighing machine 35, the tap analysis device 5 and the sterilizer 34 are disposed along the conveyance path of the conveyance device 11. The egg sorting system 100 includes the weighing machine 35 for measuring the weight of an egg. The weighing machine 35 is disposed near the trailing end of the conveyance path of the conveyance device 11. The broken egg detection device 41 and the egg image analysis device 42 will be described later in detail.

The egg sorting system 100 includes a distribution device 12 for linearly conveying eggs. The distribution device 12 is provided with a carrier and coveys the eggs having been weighed by the weighing machine 35 by the carrier. The carrier, for example, is a bucket for individually containing an egg and moving it, or is a finger and an arm for individually grabbing and carrying an egg. The distribution device 12 is connected to a control device 37 for controlling the distribution device 12. The control device 37 can specify the location of each of the eggs and can discharge an egg outside the carrier by opening the bottom of the bucket containing the egg or operating the finger grabbing the egg.

The egg sorting system 100 includes a transmitted light analysis device 6. The transmitted light analysis device 6 irradiates each of the eggs conveyed by the distribution device 12 with light and determines the state of the interior of each of the eggs based on the light transmitted through the egg. For example, the transmitted light analysis device 6 includes a deep learning model trained by using training data including information indicating the interior state of an egg and transmitted light data indicating the light transmitted through the egg. The transmitted light analysis device 6 acquires transmitted light data indicating light transmitted through an egg, determines the interior state of the egg based on the acquired transmitted light data by the deep learning model and decides a classification of the egg depending on the determination result. For example, the transmitted light analysis device 6 determines whether or not the egg is an interior abnormal egg such as an egg containing blood, an egg with broken yolk, a Yolk-less egg or a rotten egg or the like. Furthermore, the transmitted light analysis device 6 determines the type of abnormality of the egg or the degree of abnormality, for example.

In the present embodiment, the travel amount detection device 36, the egg image analysis device 42, the size measurement device 33, the tap analysis device 5, the weighing machine 35, the transmitted light analysis device 6 and the control device 37 are used to sort multiple eggs conveyed by the conveyance device 11 and the distribution device 12. The travel amount detection device 36, the egg image analysis device 42, the size measurement device 33, the tap analysis device 5, the weighing machine 35, the transmitted light analysis device 6 and the control device 37 are connected to one another. The egg image analysis device 42, the tap analysis device 5 and the transmitted light analysis device 6 determine classifications into which each of the eggs is to be sorted based on the determination result. For example, the classifications correspond to groups of eggs having different destinations. For example, the egg image analysis device 42 sorts eggs depending on the dirt on the eggshells, the tap analysis device 5 sorts eggs depending on the damage of the eggshell, and the transmitted light analysis device 6 sorts eggs depending on the interior abnormalities. The egg image analysis device 42, the tap analysis device 5 and the transmitted light analysis device 6 output information indicating the respective classifications of eggs.

The egg sorting system 100 includes multiple conveyors disposed to intersect the distribution device 12. FIG. 1 illustrates conveyors 13 and 14 disposed to intersect the distribution device 12. The conveyors disposed to intersect the distribution device 12 correspond to the respective classifications. For example, eggs sorted as being shipped to general stores are conveyed by the conveyor 13 while eggs sorted as being shipped to one except for the general stores due to damage on the eggshells or the like are conveyed by the conveyor 14. Three or more conveyors disposed to intersect the distribution device 12 may be possible. There may be a conveyor through which eggs not to be shipped are conveyed. The control device 37 accepts information from at least one of the egg image analysis device 42, the size measurement device 33, the tap analysis device 5, the weighing machine 35 and the transmitted light analysis device 36, specifies the location of each of the eggs and moves each of the eggs to the conveyor corresponding to the classifications of each of the eggs by controlling the distribution device 12. As such, eggs are sorted. In addition, the egg sorting system 100 may sort eggs to be shipped to general stores by grades of the eggs (for example, the color of the eggshells, the evenness of a color, the surface properties of the eggshells, the patterns of the eggshells, or the like).

The eggs delivered from the distribution device 12 are assumed to be packaged in a package on the conveyor 13 and shipped. Above the conveyor 13, an inside-package-image analysis device 43 is disposed. Additionally, above the conveyor 13, a label dispenser may be disposed having a function of putting a label describing information on egg such as an expiration date or the like in the package or sealing the label on the package or an each of the eggs.

The inside-package-image analysis device 43 shoots an image of eggs contained in the package and determines the state of the eggs contained in the package by image analysis. For example, the inside-package-image analysis device 43 determines based on the image the presence or absence of dirt on the eggshell of each of the eggs in the package, the degree of dirt, the type of dirt, the presence or absence of damage on the eggshell or the degree of damage. The inside-package-image analysis device 43 determines the classification into which the eggs in the package are to be sorted depending on the determined state of the eggs. The multiple eggs contained in a single package are sorted to the same classification by each package. For example, if an egg not to be shipped due to dirt or damage of the eggshell is contained in the package, the eggs contained in the package are sorted as eggs not to be shipped. The inside-package-image analysis device 43 outputs information indicating the classification for each of the packages. The inside-package-image analysis device 43 will be described in detail later.

The egg sorting system 100 includes an input device 38 to which information related to sorting of eggs is input. For example, the input device 38 includes an operation unit such as a keyboard or the like to be operated by the user and receives information by the user operating the operation unit. For example, the input device 38 includes a communication interface and receives information through the communication interface. For example, the input device 38 is connected to a communication network (not illustrated) outside the egg sorting system 100 and receives information through the communication network. The input device 38 is connected to the egg image analysis device 42, the tap analysis device 5, the transmitted light analysis device 6 and the inside-package-image analysis device 43.

The input device 38 receives information for designating the quality of eggs to be shipped. For example, information designating quality depending on the state of the eggs collected from a production site is input to the input device 38. Furthermore, information designating quality responding to a request from a customer, for example, is input to the input device 38. The input device 38 outputs the received information to the egg image analysis device 42, the tap analysis device 5, the transmitted light analysis device 6 and the inside-package-image analysis device 43. The egg image analysis device 42, the tap analysis device 5, the transmitted light analysis device 6 and the inside-package-image analysis device 43 receive information output from the input device 38 and adjust criteria for deciding a classification of eggs according to the received information. For example, if there is a specific tendency for the state of the collected eggs, the criteria for sorting the eggs having such a tendency is used. For example, if there are many eggs required for a specific application, the criterion is adjusted to ensure more eggs used for this application. The criterion is adjusted depending on the level of the required quality, for example.

Furthermore, the input device 38 may receive information related to an elapsed time from when eggs were laid. For example, the input device 38 receives information indicating an estimation value of an elapsed time from when eggs were laid to when the eggs are carried in the egg sorting system 100. Or, for example, the input layer 38 receives information indicating a lighting management time of poultry raised in a production site and a time when eggs are carried in the egg sorting system 100. The poultry have high light sensitivity and an egg-laying time is closely associated with the management of lighting and food taking. Accordingly, the egg-laying timing of the poultry can be adjusted to some extent by the management of lighting. Namely, the egg-laying time can be estimated by the cycles of light and shade. The input device 38 calculates an estimation value of the egg-laying time from the time of illumination indicated by the input information, and calculates an estimation value of an elapsed time from when eggs are laid to when the eggs are carried in based on the estimation value of the egg-laying time and the time when the egg are carried in.

The input device 38 outputs the information indicating a time elapsed from when eggs were laid to the egg image analysis device 42, the tap analysis device 5, the transmitted light analysis device 6 and the inside-package-image analysis device 43. Depending on the elapsed time from when eggs were laid, images of photographed eggs, phenomena caused by tapping the eggs and light transmitted through the eggs are slightly varied. The egg image analysis device 42, the tap analysis device 5, the transmitted light analysis device 6 and the inside-package-image analysis device 43 accept information output from the input device 38 and adjusts the criteria for deciding the classification of the eggs based on the accepted information.

The configuration of the egg sorting system 100 illustrated in FIG. 1 is one example, and the configuration of the egg sorting system 100 is not limited thereto. The multiple devices included in the egg sorting system 100 may be disposed in an order different from that illustrated in FIG. 1. The egg sorting system 100 needs not be provided with all the devices illustrated in FIG. 1. For example, the egg sorting system 100 needs not be provided with the washer 31 and the dryer 32. The eggs sorted by the egg sorting system 100 may be stored without being shipped.

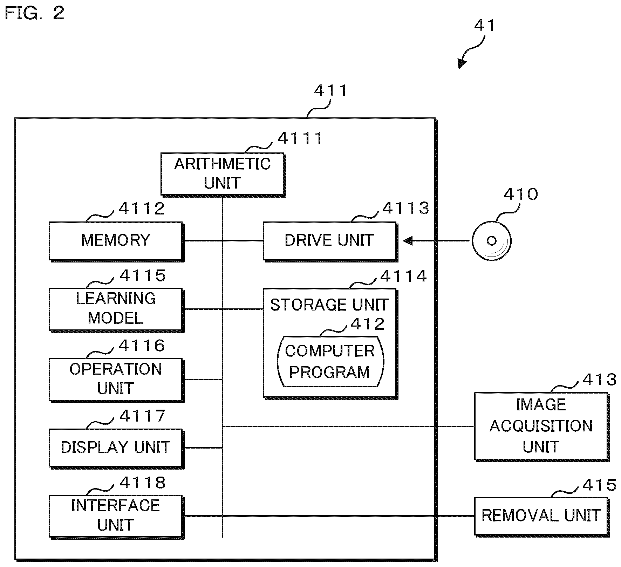

In the present embodiment, the broken egg detection device 41, the egg image analysis device 42 and the inside-package-image analysis device 43 correspond to an egg sorting device. The broken egg detection device 41, the egg image analysis device 42 and the inside-package-image analysis device 43 each execute an egg sorting method. First, the broken egg detection device 41 will be described. FIG. 2 is a block diagram illustrating an example of the functional configuration of the broken egg detection device 41. The broken egg detection device 41 is provided with an analysis unit 411 for performing information processing and an image acquisition unit 413 for acquiring an image obtained by photographing an egg conveyed by the conveyance device 11. The image acquisition unit 413 is connected to the analysis unit 411.

The analysis unit 411 is configured by using a computer. The analysis unit 411 is provided with an arithmetic unit 4111, a memory 4112 storing transitory data generated in accordance with arithmetic, a drive unit 4113 reading information from a recording medium 410 such as an optical disk or the like and a nonvolatile storage unit 4114 such as a hard disk or the like. The arithmetic unit 4111 is configured by using a central processing unit (CPU), a graphics processing unit (GPU) or a multi-core CPU. Alternatively, the arithmetic unit 4111 may be configured by using a quantum computer. The memory 4112 is a random access memory (RAM), for example. Furthermore, the analysis unit 411 includes a learning model 4115 used by determining the state of an egg according to image data representing an image obtained by photographing an egg. In addition, the analysis unit 411 is provided with an operation unit 4116 for accepting an operation from the user, a display unit 4117 for displaying an image and an interface unit 4118. The interface unit 4118 is connected to the travel amount detection device 36. The analysis unit 411 inputs and outputs information to and from the travel amount detection device 36 through the interface unit 4118.

The arithmetic unit 4111 causes the drive unit 4113 to read a computer program 412 recorded in the recording medium 410 and store the read computer program 412 in the storage unit 4114. The arithmetic unit 4111 executes processing according to the computer program 412 stored in the storage unit 4114. It is noted that the computer program 412 may be downloaded from outside of the analysis unit 411. In this case, the analysis unit 411 may not be provided with the drive unit 4113.

The learning model 4115 is a trained learning model for performing processing of determining the state of an egg according to the image data. The learning model 4115 is achieved by the arithmetic unit 4111 executing the information processing according to the computer program 412. Alternatively, the learning model 4115 may be configured to include a processor and a memory storing a necessary program and data. The learning model 4115 may be achieved by using a quantum computer.

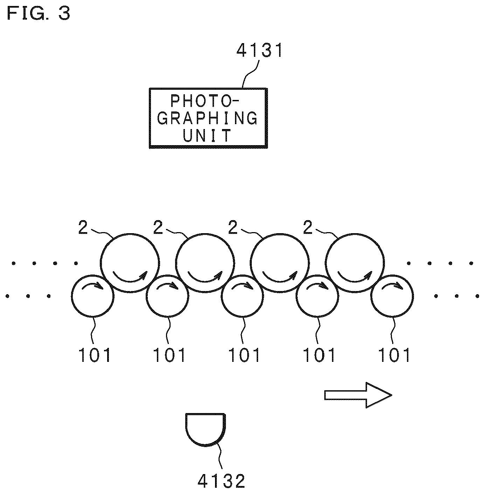

FIG. 3 is a schematic view illustrating an example of the configuration of the image acquisition unit 413. The image acquisition unit 413 includes an illumination unit 4132 illuminating eggs 2 conveyed by the conveyance device 11 and a photographing unit 4131 photographing the illuminated eggs 2. The illumination unit 4132 is configured by using a light source such as a light emitting diode (LED) or the like. The illumination unit 4132 emits light and irradiates the eggs 2 with light. For example, the illumination unit 4132 irradiates the eggs 2 with infrared rays. The photographing unit 4131 is configured by using an image sensor. The illumination unit 4132 is disposed below the conveyed eggs 2, for example. The photographing unit 4131 is disposed above the conveyed eggs 2, for example. The photographing unit 4131 photographs the eggs 2 by using light transmitted through the eggs 2.

The conveyance direction of the eggs 2 is a right-left direction in FIG. 3. The direction of conveying the eggs 2 is illustrated by a white arrow. The eggs 2 are placed on the multiple rollers 101 included in the conveyance device 11. The rollers 101 are rotated. The rollers 101 are rotated to thereby rotate the eggs 2. In FIG. 3, the direction in which the rollers 101 and the eggs 2 are rotated is indicated by arrows. The eggs 2 are illuminated by the illumination unit 4132 and photographed by the photographing unit 4131 while conveyed by the conveyance device 11. The photographed image contains the multiple eggs 2. The photographing unit 4131 photographs the eggs 2 that is being conveyed multiple times. In other words, the eggs 2 are photographed multiple times while being conveyed. In addition, the eggs 2 are photographed multiple times while being rotated. Thus, multiple portions of the eggs 2 about the axis of rotation are photographed.

The image acquisition unit 413 is configured to photograph a large number of eggs 2 that are being conveyed while aligned in multiple rows. For example, the photographing unit 4131 simultaneously photographs multiple eggs 2 included in multiple rows. The photographed image contains the eggs 2 in the multiple rows. The image acquisition unit 413 may photograph one egg 2 by one photographing unit 4131, or may be provided with multiple photographing units 4131 and photograph one egg 2 by the multiple photographing units 4131 multiple times. The image acquisition unit 413 may include an optical component (not illustrated) such as a mirror, a lens and optical filter or the like. The image acquisition unit 413 may utilize light from outside without being provided with the illumination unit 4132.

The photographing unit 4131 creates image data representing the image of the eggs 2 obtained by photographing eggs 2. Here, the photographing unit 4131 creates image data representing a monochrome image. As such, the image acquisition unit 413 acquires the image of the eggs 2 and inputs the image data representing the image of the eggs 2 to the analysis unit 411. The image acquisition unit 413 corresponds to an image data acquisition unit. Note that the image acquisition unit 413 may be configured to acquire an image obtained by photographing eggs 2 using reflected light reflected by the eggs 2 or fluorescence. Furthermore, the image acquisition unit 413 may be configured to acquire two or more images out of the image obtained by photographing the eggs 2 with reflected light, the image obtained by photographing the eggs 2 with transmitted light and the image obtained by photographing the eggs 2 with fluorescence.

The analysis unit 411 performs processing of determining the state of each of the eggs 2 based on the image data and determining a classification of each of the eggs 2 depending on the determination result. The state of the egg 2 to be determined is specifically the presence or absence of damage of the eggshell or the size of the damage. Depending on the presence or absence of damage of the eggshell and the size of the damage, images obtained by photographing eggs 2 are different. This makes it possible to determine the state of the eggs 2 according to the image data. Note that the egg sorting system 100 includes a removal unit 415 that removes an egg that is determined to be a broken egg. The removal unit 415 has a mechanism for removing an egg 2 that is being conveyed by the conveyance device 11 from the conveyance device 11. For example, the conveyance path of the conveyance device 11 is provided with a hole. The removal unit 415 is configured to open or close the hole on the conveyance path. If the removal unit 415 opens the hole of the conveyance path, an egg 2 falls through the hole and is removed from the conveyance device 11.

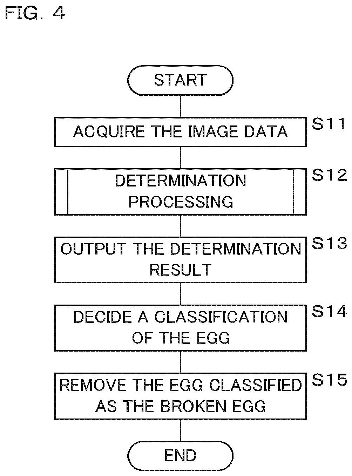

FIG. 4 is a flowchart showing a processing procedure of sorting eggs 2 performed by the analysis unit 41 of the broken egg detection device 41. The step is abbreviated as S below. The arithmetic unit 4111 of the analysis unit 411 executes the following processing according to the computer program 412. The analysis unit 411 accepts information from the travel amount detection device 36 through the interface unit 4118, and the arithmetic unit 4111 specifies a location of each of the eggs 2 based on the information from the travel amount detection device 36. The arithmetic unit 4111 acquires the image data for one egg 2 of which the location is specified (S11). The arithmetic unit 4111 stores the image data in the storage unit 4114.

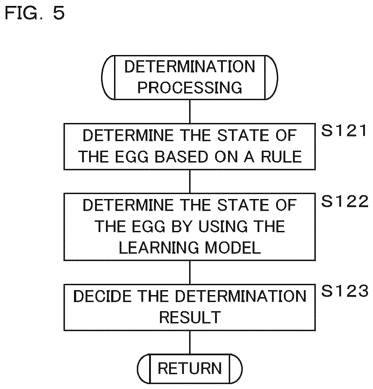

The arithmetic unit 4111 next performs determination processing for determining the state of the egg 2 based on the image data (S12). FIG. 5 is a flowchart showing the procedure for the determination processing. The arithmetic unit 4111 determines the state of the egg 2 based on a rule (S121). At step S121, the arithmetic unit 4111 determines the presence or absence of damage of the eggshell or the size of damage according to the image data based on a predefined rule. The arithmetic unit 4111 may perform processing of determining the state of the egg 2 by a conventionally known method. For example, the arithmetic unit 4111 detects damage of the eggshell by performing edge detection of the image of the egg 2 and determines the size of the damage of the eggshell by comparing the size of the damage included in the image with a predetermined threshold. The processing at step S121 corresponds to a second determination unit.

The arithmetic unit 4111 then determines the state of the egg 2 by using the learning model 4115 (S122). The learning model 4115 is trained in advance by using the training data including the image data representing the images of photographed eggs 2 and the information indicating the presence or absence of damage of the eggshells of the eggs 2 or the size of damage so as to determine the presence or absence of damage or the size of damage of the eggshell of an egg 2 from an image data of the egg2. For example, the learning model 4115 employs a neural network.

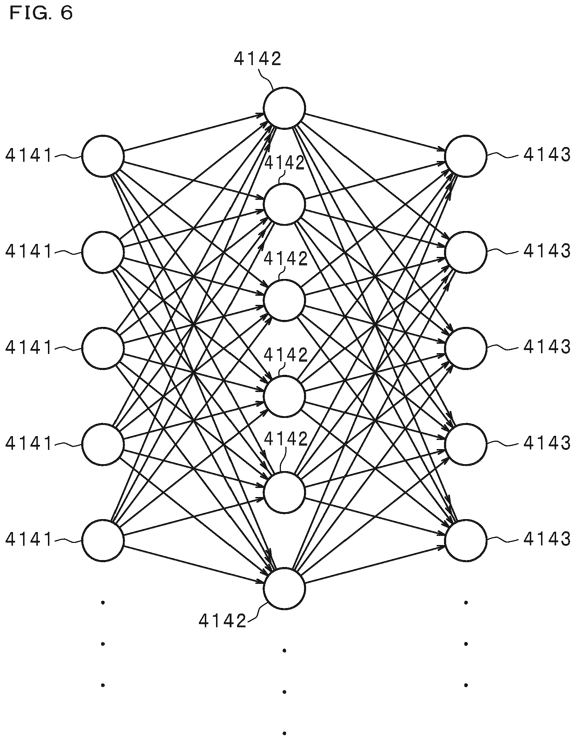

FIG. 6 is a conceptual diagram illustrating an example of the functional configuration of the learning model 4115. The learning model 4115 employs a neural network including an input layer, an intermediate layer and an output layer each having multiple nodes. The input layer has multiple nodes 4141 to which image data is input. For example, each pixel value of the pixels included in an image is input to any of the nodes 4141. The intermediate layer has multiple nodes 4142 each performing arithmetic on the data input from each of the nodes 4141 of the input layer by using parameters. The multiple nodes 4142 respectively output data to multiple nodes 4143 included in the output layer. The output layer has the multiple nodes 4143 each accepting data on which arithmetic is performed from each of the nodes 4142 of the intermediate layer and outputting the state of the egg 2. For example, one of the nodes 4143 outputs a probability of the presence of damage of the eggshell as a score while another one of the nodes 4143 outputs a probability of the presence of damage with a certain size as a score. Though FIG. 6 shows an example in which the intermediate layer includes a single layer, the intermediate layer may include multiple layers. The learning model 4115 may employ a convolutional neural network (CNN) or a recurrent neural network (RNN) as a neural network.

The learning model 4115 is trained in advance by using training data. As to a large number of eggs 2 the states of which have already been identified and which include eggs 2 having the eggshells with no damage and no dirt, image data are acquired to create training data. The training data includes information indicating the state of each of the eggs 2 and the image data representing the image of the eggs 2 are included. Adjustment of an arithmetic parameter of each of the nodes is trained such that when the image data as to each of the eggs 2 included in the training data is input to each of the nodes 4141 of the input layer, each of the nodes 4143 of the output layer outputs a score corresponding to the state of each of the eggs 2 indicated by the information included in the training data. For example, in the training, an error is calculated by using an error function regarding the output value from each of the nodes 4143 in the output layer and the output value predicted from the training data as variables. The arithmetic parameter of each of the nodes is adjusted such that the error is minimized by a back propagation.

Training of the learning model 4115 may be performed by the broken egg detection device 41. For example, the image acquisition unit 413 acquires the image data of eggs 2 the state of which have already been identified to create training data, and the analysis unit 411 executes information processing for training. Alternatively, training data may be input from outside the broken egg detection device 41, and the analysis unit 411 may execute information processing for training. Alternatively, training is performed by a training device located outside the broken egg detection device 41, and the learning model 4115 may be created based on the training result. The training device is configured by using a computer. Image data of an egg 2 is acquired, the state of the egg 2 after the image data has been acquired is checked, and training data including the image data and the information indicating the checked state of the egg 2 may be created. The training device may receive an input of the training data to perform training. The training device may receive the image data and the information indicating the state of the egg 2, create training data including the input data and the information and perform training by using the created training data.

The learning model 4115 may be configured to receive an input of the image data representing an image obtained by photographing an egg 2 by using reflected light or fluorescence and output the state of the egg 2. Alternatively, the learning model 4115 may include two or more learning models out of a learning model to which image data representing an image obtained by photographing the egg 2 using reflected light is to be input, a learning model to which image data representing an image obtained by photographing the egg 2 using transmitted light is to be input, and a learning model to which image data representing an image obtained by photographing the egg 2 using fluorescence is to be input.

The arithmetic unit 4111 determines the state of the egg 2 according to the output from the learning model 4115. For example, the arithmetic unit 4111 determines that the eggshell has no damage if the probability of presence of damage on the eggshell is less than a predetermined threshold, and determines that the eggshell has damage if the probability of presence of damage is equal to or larger than the predetermined threshold. For example, the arithmetic unit 4111 determines the size of the damage having the maximum score value to be the size of the damage of the eggshell. Furthermore, the arithmetic unit 4111 may determine the state of the egg 2 by a combination of two or more outputs out of the output of the learning model to which the image data representing an image obtained by photographing the egg 2 using the reflected light is input, the output of the learning model to which the image data representing an image obtained by photographing the egg 2 using the transmitted light is input, and the output of the learning model to which the image data representing an image obtained by photographing the egg 2 using the fluorescence is input.

The arithmetic unit 4111 decides the determination result of the state of the egg 2 based on the determination result obtained at S121 and the determination result obtained at S122 (S123). At S123, the determination result of the egg 2 is decided by a combination of the rule-based determination result obtained at S121 and the determination result using the learning model 4115 obtained at S122. For example, assuming that it is possible to determine that the state of the egg 2 is unknown at S121, if the determination result at S121 is not unknown, the arithmetic unit 4111 adopts the determination result obtained at S121 at S123, and if the determination result at S121 is unknown, the arithmetic unit 4111 performs the processing at S122 and adopts the determination result obtained at S122 at S123. Alternatively, the arithmetic unit 4111 compares the score obtained by the processing at S122 with a predetermined threshold and decides which one of the determination results obtained at S121 and S122 is to be adopted in accordance with the comparison result. Alternatively, the arithmetic unit 4111 first performs the processing at S122, for example. If the state of the egg 2 is unknown at S122 such as a case where the value of the score is less than a predetermined threshold, for example, the arithmetic unit 4111 performs the processing at S121 and adopts the determination result obtained at S121 at S123. If the state of the egg 2 is not unknown at S122, for example, the arithmetic unit 4111 adopts the determination result obtained at S122 at S123. For example, the arithmetic unit 4111 generates scores indicating the probabilities of the state of the egg 2 being respective states at S121 and S122, calculates a combined score obtained by a combination of predetermined calculation methods such as summing, average calculation and weighted average calculation of the scores obtained at step S121 and S122, and decides a determination result based on the combined score. After completion of S123, the arithmetic unit 4111 returns the processing to the main processing. The combined use of the rule-based determination and the determination using the learning model 4115 enables suitable determination. Note that only the determination using the learning model 4115 may be performed without performing the rule-based determination in the processing at step S12.

The arithmetic unit 4111 outputs the determination result of the state of the eggs 2 (S13). At step S13, the arithmetic unit 4111 displays an image including the determination result on the display unit 4117. For example, the arithmetic unit 4111 displays the number of eggs 2 with the eggshells having no damage, the number of eggs 2 with the eggshells having damage or the number of eggs 2 with the eggshells having the degree of damage falling within a predetermined range is displayed on the display unit 4117. The arithmetic unit 4111 may display both of the rule-based determination result obtained at step S121 and the determination result using the learning model 4115 obtained at step S122 on the display unit 4117.

The arithmetic unit 4111 then decides a classification of the egg 2 depending on the determination result of the state of the egg 2 (S14). For example, the arithmetic unit 4111 decides a classification of the egg 2 in which the size of damage of the eggshell is above a predetermined criterion, a classification of the egg 2 in which the eggshell has no damage and a classification of the egg 2 in which the size of damage of the eggshell is equal to or lower than a predetermined criterion. The processing at S14 corresponds to a decision unit.

The arithmetic unit 4111 removes the egg 2 classified as the broken egg from the conveyance device 11 (S15). At S15, the arithmetic unit 4111 specifies the location of the egg 2 classified as the broken egg and causes the removal unit 415 to remove the egg 2 classified as the broken egg. For example, the removal unit 415 opens a hole on the conveyance path at a timing when the egg 2 classified as the broken egg passes over the hole of the conveyance path. The egg 2 classified as the broken egg falls through the hole and is removed from the conveyance device 11. Removal by the removal unit 415 is not performed on an egg 2 not classified as the broken egg. The analysis unit 411 performs the processing at S11 to S15 on each of the multiple eggs 2 conveyed by the conveyance device 11. The analysis unit 411 may perform the processing as to the multiple eggs 2 sequentially or in parallel. Note that the broken egg detection device 41 may perform processing of outputting classification data indicating the classification of the egg 2 through the interface unit 4118 to outside the broken egg detection device 41 without performing the processing at S15. The control device 37 may acquire the output classification data, specify the egg 2 classified as the broken egg based on the classification data and sort the egg 2.

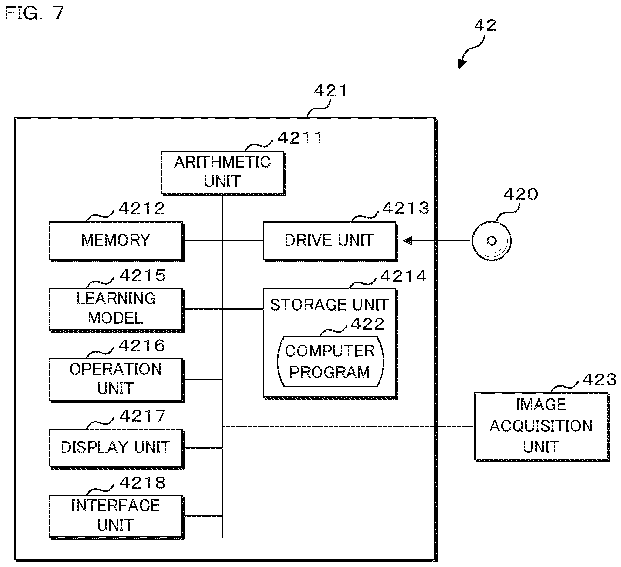

Next, the egg image analysis device 42 will be described. FIG. 7 is a block diagram illustrating an example of the functional configuration of the egg image analysis device 42. The egg image analysis device 42 is provided with an analysis unit 421 for performing information processing and an image acquisition unit 423 for acquiring an image obtained by photographing an egg 2 conveyed by the conveyance device 11. The image acquisition unit 423 is connected to the analysis unit 421.

The analysis unit 421 is configured by using a computer. The analysis unit 421 is provided with an arithmetic unit 4211, a memory 4212, a drive unit 4213 and a nonvolatile storage unit 4214. The arithmetic unit 4211 may be configured by using a CPU, a GPU, a multicore CPU or a quantum computer, for example. The memory 4212 is a RAM, for example. Furthermore, the analysis unit 421 is provided with a learning model 4215 to be used for determining the state of an egg 2 according to the image data representing an image of the egg 2.

Moreover, the analysis unit 421 is provided with an operation unit 4216 for accepting an operation from the user, a display unit 4217 for displaying an image and an interface unit 4218. The operation unit 4216 accepts information such as a text or the like by accepting an operation from the user. The operation unit 4216 is a touch panel, a keyboard or a pointing device, for example. The display unit 4217 is a liquid crystal display or an electroluminescent (EL) display. Furthermore, the analysis unit 421 may be provided with a communication unit and may have a function of accepting information from an operation unit of a smartphone or the like outside the analysis unit 421 through the communication unit and displaying an image on the display unit of the smartphone or the like outside the analysis unit 421. The interface unit 4218 is connected to the travel amount detection device 36 and the input device 38. The analysis unit 421 inputs and outputs information to and from the travel amount detection device 36 and the input device 38 through the interface unit 4218.

The arithmetic unit 4211 causes the drive unit 4213 to read a computer program 422 recorded in a recording medium 420 such as an optical disk or the like and store the read computer program 422 in the storage unit 4214. The arithmetic unit 4211 executes processing according to the computer program 422 stored in the storage unit 4214. For example, the arithmetic unit 4211 loads the computer program 422 from the storage unit 4214 onto the memory 4212 as necessary and the arithmetic unit 4211 executes necessary processing for the analysis unit 421 according to the loaded computer program 422. Note that the computer program 422 may be downloaded from outside of the analysis unit 421. Here, the analysis unit 421 may not be provided with the drive unit 4213.

The learning model 4215 is a trained learning model for performing processing of determining the state of an egg 2 according to the image data. The learning model 4215 is achieved by the arithmetic unit 4211 executing information processing according to the computer program 422. Alternatively, the learning model 4215 may be configured to include a processor and a memory storing a necessary program and data. The learning model 4215 may be achieved by using a quantum computer.

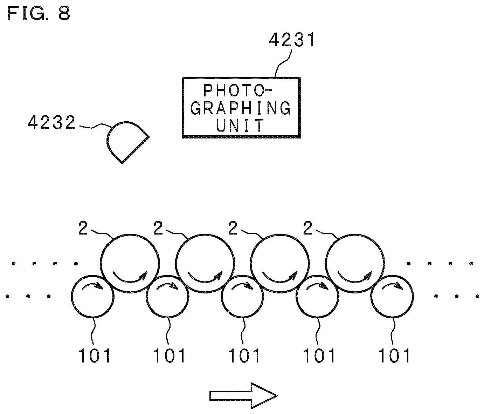

FIG. 8 is a schematic view illustrating an example of the configuration of the image acquisition unit 423. The image acquisition unit 423 includes an illumination unit 4232 illuminating eggs 2 conveyed by the conveyance device 11 and a photographing unit 4231 photographing the illuminated eggs 2. The illumination unit 4232 is configured by using a light source such as a fluorescent lamp, an LED or the like. The illumination unit 4232 emits light and irradiates the eggs 2 with light. The photographing unit 4231 is configured by using an image sensor and shoots a color image. The illumination unit 4232 and the photographing unit 4231 are disposed above the eggs 2 that are being conveyed, for example. The photographing unit 4231 photographs the eggs 2 by using a reflected light reflected by the eggs 2.

The conveyance direction of the eggs 2 is a right-left direction in FIG. 8. The direction of conveying the eggs 2 is illustrated by a white arrow. The illumination unit 4232 and the photographing unit 4231 are disposed at positions above the eggs 2. FIG. 8 illustrates rollers 101 included in the conveyance device 11. The eggs 2 are placed on the multiple rollers 101. The rollers 101 are rotated. The rollers 101 are rotated to thereby rotate the eggs 2. In FIG. 8, the directions in which the rollers 101 and the eggs 2 are rotated are indicated by arrows. The eggs 2 are illuminated by the illumination unit 4232 and photographed by the photographing unit 4231 while conveyed by the conveyance device 11. The photographed image contains multiple eggs 2. The photographing unit 4231 photographs the eggs 2 that are being conveyed multiple times. Namely, the eggs 2 are photographed multiple times while being conveyed. Moreover, the eggs 2 are photographed multiple times while being rotated. Thus, multiple portions of the eggs 2 about the axis of rotation are photographed.

The image acquisition unit 423 is able to photograph a large number of eggs 2 that are being conveyed while aligned in multiple rows. For example, the photographing unit 4231 simultaneously photographs multiple eggs 2 included in multiple rows. The photographed image contains the eggs 2 in multiple rows. Alternatively, the image acquisition unit 423 may photograph one egg 2 by a single photographing unit 4231, or may photograph one egg 2 multiple times by multiple photographing units 4231. The image acquisition unit 423 may include an optical component (not illustrated) such as a mirror, a lens, an optical filter or the like. The image acquisition unit 423 may utilize light from outside without being provided with the illumination unit 4232.

The photographing unit 4231 creates image data representing an image obtained by photographing the eggs 2. Here, the photographing unit 4231 creates the image data representing a color image. As such, the image acquisition unit 423 acquires the image of the eggs 2 and inputs the image data representing the image of the eggs 2 to the analysis unit 421. The image acquisition unit 423 corresponds to an image data acquisition unit. Note that the image acquisition unit 423 may be configured to acquire an image obtained by photographing the eggs 2 using transmitted light transmitted through the eggs 2 or an image obtained by photographing the eggs 2 using the excited fluorescence of the eggs 2. Furthermore, the image acquisition unit 423 may be configured to acquire two or more images out of the image obtained by photographing the eggs 2 using reflected light, the image obtained by photographing the eggs 2 using transmitted light and the image obtained by photographing the eggs 2 using fluorescence.

The analysis unit 421 performs processing of determining the state of each of the eggs 2 based on the image data and deciding a classification of each of the eggs 2 according to the determination result. The state of each of the eggs 2 to be determined specifically includes a color of the eggshell, evenness of a color, the presence or absence of dirt on the eggshell, the degree of dirt, the type of dirt, the presence or absence of damage of the eggshell, the degree of damage, the presence or absence of a deformity of the eggshell, the type of a deformity, surface properties of the eggshell, a pattern of the eggshell or a printing on the eggshell. The color of the eggshell includes white, light brown, brown or the like. The degree of dirt means the size of dirt, the depth of a color or the like on the egg 2. The type of dirt includes spotted yellowish dirt, brown dirt, white dirt, gray dirt, black dirt, or a speck of dirt spread like a stain. The deformity of the eggshell means that the shape of the eggshell is different from a standard shape and includes a jumbo-sized egg, a mini-sized egg or the like. The surface properties of the eggshell includes a smooth surface of the eggshell, a ridge or roughness on the surface of the eggshell or the like. The pattern of the eggshell includes a plain color, a freckle-like pattern, a dotted pattern or the like. The image data is different depending on the state of the eggs 2. This makes it possible to determine the state of each of the eggs 2 according to the image data.

Conventionally, a technique for determining the state of an egg 2 according to the image data has widely been known. A color of the eggshell, the presence or absence of dirt on the eggshell, the degree of dirt or the type of dirt is determined based on saturation or brightness of image data or a distribution of the pixels of each of the colors on the egg 2. Similarly, the presence or absence of damage of the eggshell, the degree of damage, the evenness of a color of the eggshell, the surface properties of the eggshell, the pattern of the eggshell or the printing on the eggshell can also be determined from the distribution of each of the colors. In addition, the presence or absence of a deformity of the eggshell or the type of a deformity can be determined from the shape of the eggshell included in the image. As such, the state of each of the eggs 2 can be determined according to the image data.

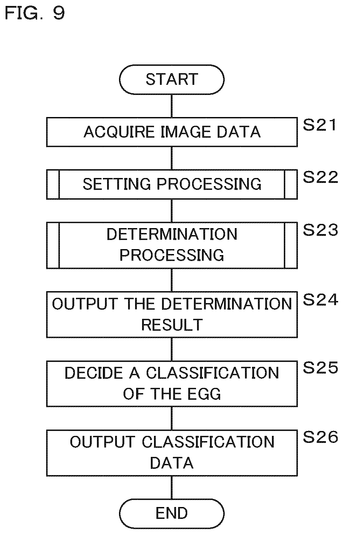

The analysis unit 421 of the egg image analysis device 42 performs processing of sorting eggs 2. FIG. 9 is a flowchart showing a processing procedure for sorting eggs 2. The arithmetic unit 4211 of the analysis unit 421 executes the following processing according to the computer program 422. The analysis unit 421 accepts information from the travel amount detection device 36 through the interface unit 4218, and the arithmetic unit 4211 specifies a location of each of the eggs 2 based on the information from the travel amount detection device 36. The arithmetic unit 4211 acquires image data for any one of the eggs 2 the location of which is specified from the image acquisition unit 423 (S21). At step S21, the arithmetic unit 4211 obtains the image data from the photographing unit 4231 that photographs the egg 2 the location of which has been specified and acquires image data related to multiple times of photographing from each of the photographing units 4231. Namely, the arithmetic unit 4211 acquires the image data of multiple times of photographing for a single egg 2. The arithmetic unit 4211 stores the image data in the storage unit 4214. The arithmetic unit 4211 then performs setting processing of setting for sorting eggs 2 (S22). The detail of the setting processing will be described below. The arithmetic unit 4211 may execute the processing at S21 and S22 in a reverse order.

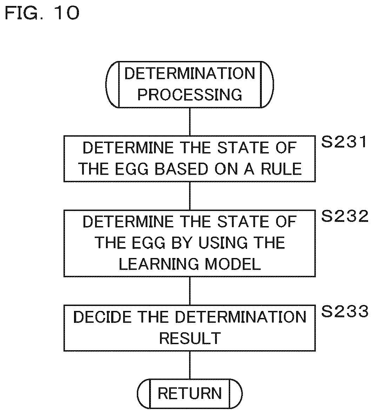

The arithmetic unit 4211 next performs determination processing for determining the state of each of the eggs 2 based on the image data (S23). FIG. 10 is a flowchart showing the procedure for the determination processing. The arithmetic unit 4211 determines the state of each of the eggs 2 based on a rule (S231). At step S231, the arithmetic unit 4211 determines the state of each of the eggs 2 according to the image data based on a predefined rule. The arithmetic unit 4211 may perform processing of determining the state of each of the eggs 2 by a conventionally known method. For example, the arithmetic unit 4211 determines a color of the eggshell of the egg 2, evenness of the color of the eggshell, the presence or absence of dirt on the eggshell, the degree of dirt, the type of dirt, the presence or absence of damage of the eggshell, the degree of damage, surface properties of the eggshell, a pattern of the eggshell or a printing on the eggshell based on the distribution of pixel values of R, G and B in the image. For example, the arithmetic unit 4211 determines the presence or absence of deformity of the eggshell of the egg 2 or the type of deformity based on the shape of the eggshell included in the image. The arithmetic unit 4211 may use all the image data of multiple times of photographing or a single time of photographing. The processing at step S231 corresponds to a second determination unit.

The arithmetic unit 4211 next determines the state of each of the eggs 2 by using the learning model 4215 (S232). The learning model 4215 is trained in advance by using training data including image data indicating images of photographed egg 2 and information indicating a color of the eggshells, evenness of the color, the presence or absence of dirt on the eggshells, the degree of dirt, the type of dirt, the presence or absence of damage of the eggshells, the degree of damage, the presence or absence of a deformity of the eggshells, the type of a deformity, surface properties of the eggshells, a pattern of the eggshells or a printing on the eggshells so as to determine from the image data the color of the eggshell of the egg 2, the evenness of the color, the presence or absence of dirt on the eggshell, the degree of dirt, the type of dirt, the presence or absence of damage of the eggshell, the degree of damage, the presence or absence of a deformity of the eggshell, the type of the deformity, the surface properties of the eggshell, the pattern of the eggshell or the printing on the eggshell. For example, the learning model 4215 employs a neural network.

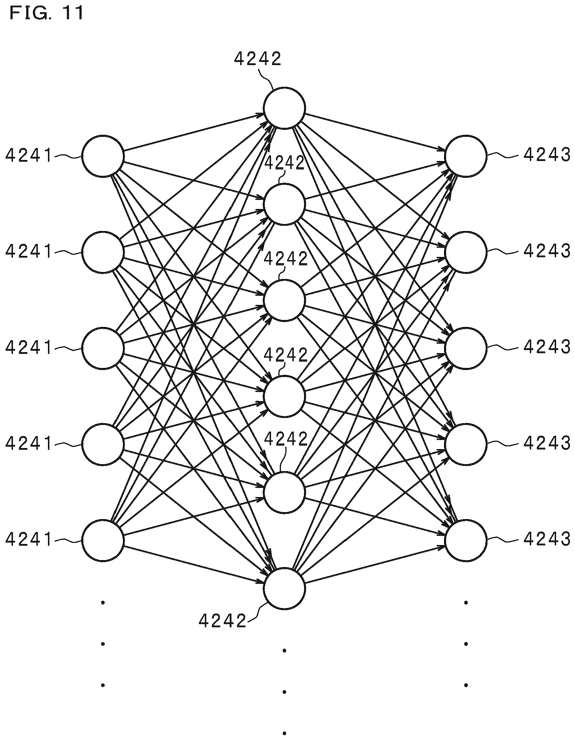

FIG. 11 is a conceptual diagram illustrating an example of the functional configuration of the learning model 4215. The learning model 4215 employs a neural network. The input layer has multiple nodes 4241 to each of which image data is input. For example, each of the image data of multiple times of photographing is input to any of the nodes 4241. At least one of the information designating quality and the information indicating an elapsed time from egg-laying may be input to the input layer. The intermediate layer has multiple nodes 4242 each performing arithmetic on the data input from each of the nodes 4241 of the input layer by using a parameter. The multiple nodes 4242 respectively output data to multiple nodes 4243 included in the output layer. The output layer has the multiple nodes 4243 each receiving data on which arithmetic has been performed from each of the nodes 4242 of the intermediate layer and outputting the state of the egg 2. For example, one of the nodes 4243 outputs a probability of the color of the eggshell of the egg 2 being a specific color as a score, another one of the nodes 4243 outputs a probability of presence of dirt on the eggshell as a score, another one of the nodes 4243 outputs a probability of the degree of dirt being a predetermined degree as a score, and another one of the nodes 4243 outputs a probability of the type of dirt being a specific type as a score. For example, each of the nodes 4243 outputs a probability of the evenness of a color of the eggshell being a specific state, a probability of presence of damage on the eggshell, a probability of the degree of damage being a predetermined degree, a probability of the eggshell having a deformity, a probability of the deformity of the eggshell being a specific type, a probability of the surface properties of the eggshell being a predetermined type, a probability of the pattern of the eggshell being a predetermined type or a probability of a predetermined printing existing on the eggshell as a score, for example. The intermediate layer may include multiple layers. The learning model 4215 may employ CNN or RNN as a neural network.

The learning model 4215 is trained in advance by using training data. As to a large number of eggs 2 the states of which have already been identified and which include eggs 2 having the eggshells with no damage and no dirt, image data are acquired to create training data. The training data includes information indicating the state of each of the eggs 2 and the image data representing the image obtained by photographing each of the eggs 2. Adjustment of an arithmetic parameter of each of the nodes is trained such that when the image data as to each of the eggs 2 included in the training data is input to each of the nodes 4241 of the input layer, each of the nodes 4243 of the output layer outputs a score corresponding to the state of each of the eggs 2 indicated by the information included in the training data. For example, in the training, an error is calculated by using an error function regarding the output value from each of the nodes 4243 in the output layer and the output value predicted from the training data as variables. The arithmetic parameter of each of the nodes is adjusted such that the error is minimized by a back propagation.

Training of the learning model 4215 may also be performed by the egg image analysis device 42. For example, the image acquisition unit 423 acquires the image data of the egg 2 the state of which has already been identified to create training data, and the analysis unit 421 executes information processing for training. Alternatively, training data may be input from outside the egg image analysis device 42, and the analysis unit 421 may execute information processing for training. Alternatively, training is performed by a training device located outside the egg image analysis device 42, and the learning model 4215 may be created based on the training result. The training device is configured by using a computer. Image data of an egg 2 is acquired, the state of the egg 2 after the image data has been acquired is checked, and training data including the image data and the information indicating the checked state of the egg 2 may be created. The training device may receive the training data and may be trained. The training device may receive the image data and the information indicating the state of the egg 2, create training data including the input data and information, and perform training by using the created training data.

The learning model 4215 may be configured to receive image data representing an image obtained by photographing an egg 2 by using transmitted light or fluorescence and to output the state of the egg 2. Alternatively, the learning model 4215 may include two or more learning models out of the learning model to which image data representing an image obtained by photographing the egg 2 using reflected light is to be input, the learning model to which image data representing an image obtained by photographing the egg 2 using transmitted light is to be input and the learning model to which image data representing an image obtained by photographing the egg 2 using fluorescence is to be input.

The arithmetic unit 4211 determines the state of an egg 2 according to the output of the learning model 4215. For example, the arithmetic unit 4211 regards a color having the maximum score as the color of the eggshell of the egg 2. For example, the arithmetic unit 4211 determines the state of the evenness of a color of the eggshell having the maximum score to be the evenness of the color of the eggshell of the egg 2. For example, the arithmetic unit 4211 determines that the eggshell has dirty if the probability of presence of dirt on the eggshell is equal to or larger than a predetermined threshold, and determines that the eggshell has no dirt if the probability of presence of dirt on the eggshell is less than the predetermined threshold. For example, the arithmetic unit 4211 determines the degree of dirt having the maximum score to be the degree of dirt on the eggshell. For example, the arithmetic unit 4211 determines a type of dirt having the maximum score to be the type of the dirt on the eggshell. For example, the arithmetic unit 4211 determines that the eggshell has a deformity if the probability of presence of a deformity of the eggshell is equal to or larger than a predetermined threshold, and determines that the eggshell has no deformity if the probability of presence of a deformity of the eggshell is less than the predetermined threshold. For example, the arithmetic unit 4211 determines a type of a deformity having the maximum score to be the type of the deformity of the eggshell. For example, the arithmetic unit 4211 determines the type of surface properties of the eggshell having the maximum score to be the surface properties of the eggshell of the egg 2. For example, the arithmetic unit 4211 determines the type of a pattern of the eggshell having the maximum score to be the pattern of the eggshell of the egg 2. The same applies to the presence, or absence of damage of the eggshell, the degree of damage and a printing on the eggshell.

Furthermore, the learning model 4215 may individually include a learning model for determining a color of the eggshell, a learning model for determining the evenness of a color of the eggshell, a learning model for determining the presence or absence of dirt on the eggshell, a learning model for determining the degree of dirt, a learning model for determining a type of the dirt on the eggshell, a learning model for determining the presence or absence of damage of the eggshell, a learning model for determining the degree of damage of the eggshell, a learning model for determining the presence or absence of a deformity of the eggshell, a learning model for determining a type of a deformity of the eggshell, a learning model for determining surface properties of the eggshell, a learning model for determining a pattern of the eggshell or a learning model for determining a printing on the eggshell. At S232, the arithmetic unit 4211 performs each of the determinations by using each of the learning models.

To the input layer of the learning model 4215, image data of multiple times of photographing may all be input or only a part of them may be input. Furthermore, the arithmetic unit 4211 may determine the state of the egg 2 by a combination of two or more outputs out of the output of the learning model to which image data representing an image obtained by photographing the egg 2 using reflected light is input, the output of the learning model to which image data representing an image obtained by photographing the egg 2 using transmitted light is input, and the output of the learning model to which image data representing an image obtained by photographing the egg 2 using fluorescence is input. The processing at step S232 corresponds to a first determination unit. The processing at S231 and S232 may be executed in the reverse order or may be executed in parallel.

The arithmetic unit 4211 then decides the determination result of the state of the egg 2 based on the determination result obtained at S231 and the determination result obtained at S232 (S233). At S233, the determination result of the egg 2 is decided by a combination of the rule-based determination result obtained at S231 and the determination result using the learning model 4215 obtained at S232. A method of combining the rule-based determination result and the determination result using the learning model is the same as the method in the processing at S123. After completion of S233, the arithmetic unit 4211 returns the processing to the main processing. The combined use of the rule-based determination and the determination using the learning model 4215 enables suitable determination. Note that only the determination using the learning model 4215 may be performed without performing the rule-based determination in the processing at step S23.

The arithmetic unit 4211 then outputs the determination result of the state of the egg 2 (S24). At S24, the arithmetic unit 4211 displays an image including a determination result on the display unit 4217. For example, the arithmetic unit 4211 displays on the display unit 4217 the numbers of eggs 2 having the eggshells with respective colors, the numbers of eggs 2 having the eggshells with respective states of the color evenness, the number of eggs 2 having the eggshell with no dirt, the number of eggs 2 having the eggshell with dirt, the number of eggs 2 having the degree of dirt falling within a predetermined range, the numbers of eggs 2 having respective types of dirt, the number of eggs 2 having the eggshells with no damage, the number of eggs 2 having the eggshells with damage, the number of eggs 2 having the degree of damage falling within a predetermined range, the number of eggs 2 having the eggshells with a deformity, the numbers of eggs 2 having the respective types of deformities, the numbers of eggs 2 having the respective types of surface properties, the numbers of eggs 2 having the respective types of patterns, or the number of eggs 2 having the eggshells with a predetermined printing. The arithmetic unit 4211 may display the rule-based determination result obtained at step S231 and the determination result using the learning model 4215 obtained at step S232 on the display unit 4217. The user can find the state of the eggs 2 that are being conveyed by the conveyance device 11 by confirming the content displayed on the display unit 4217. Furthermore, the arithmetic unit 4211 outputs the data indicating the state of the egg 2 to outside of the egg image analysis device 42 through the interface unit 4218. For example, the transmitted light analysis device 6 acquires data indicating the state of the egg 2 such as a color of the eggshell, the presence or absence of dirt or the like and performs processing using the acquired data.

The arithmetic unit 4211 next decides a classification of the egg 2 according to the determination result of the state of the egg 2 (S25). For example, the arithmetic unit 4211 sorts the egg 2 by color. If the degree of dirt on the eggshell of the egg 2 is above a predetermined criterion, for example, the arithmetic unit 4211 decides the classification of the egg 2 to be categorized in the eggs 2 different in application from the eggs 2 to be shipped to stores, such as a group of eggs 2 for processing. If there is no dirt on the eggshell or if the degree of dirt is equal to or below the criterion, for example, the arithmetic unit 4211 decides the classification of the egg 2 to be categorized in a group of eggs 2 allowed to be shipped to a general store. Furthermore, the arithmetic unit 4211 may sort eggs 2 depending on the evenness of a color of the eggshell, the type of dirt on the eggshell, the presence or absence of damage of the eggshell, the degree of damage, the presence or absence of a deformity of the eggshell, the type of a deformity, surface properties of the eggshell, a pattern of the eggshell or a printing on the eggshell. The processing at S25 corresponds to a decision unit.

The arithmetic unit 4211 then outputs classification data indicating the classification of the eggs 2 to outside of the egg image analysis device 42 through the interface unit 4218 (S26). The processing at S26 corresponds to an output unit. The control device 37 acquires the classification data, controls the distribution device 12 based on the classification data, and performs egg distribution by moving the egg 2 toward a package on the conveyor corresponding to the classification for the egg 2. Hence, the arithmetic unit 4211 ends the processing for sorting eggs 2. The analysis unit 421 performs the processing from S21 to S26 on each of the multiple eggs 2 conveyed by the conveyance device 11. The analysis unit 421 may perform the processing as to the multiple eggs 2 successively or in parallel.

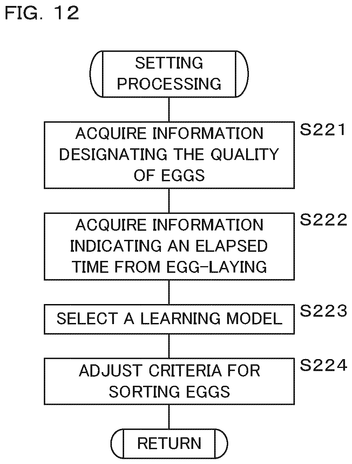

The setting processing at S22 will next be described. FIG. 12 is a flowchart showing the procedure for the setting processing. The arithmetic unit 4211 acquires information designating the quality of eggs 2 to be shipped (S221). The quality (lot information, for example) of the eggs 2 depending on the state of the collected eggs 2 is set by the input device 38. At S221, the arithmetic unit 4211 accepts the information designating the set quality from the input device 38 through the interface unit 4218. Alternatively, information designating the quality responding to a request from a customer is input to the input device 38. The arithmetic unit 421 then accepts the information designating the quality from the input device 38 through the interface unit 4218. The processing at S221 corresponds to a first acquisition unit.

The criteria for deciding a classification of eggs 2 needs to be adjusted depending on the quality of eggs 2 to be shipped. By adjustment of the criteria for deciding the classification of eggs 2, the eggs 2 can suitably be sorted so as to acquire the eggs 2 with necessary quality. Alternatively, by adjustment of the learning model 4215 used for determining the state of an egg 2 depending on the quality of eggs 2 to be shipped, the eggs 2 can suitably be sorted depending on the determined state of the egg 2.

The arithmetic unit 4211 then acquires information indicating an elapsed time from when eggs 2 were laid (S222). At S222, the arithmetic unit 4211 accepts information indicating an estimation value of an elapsed time from when eggs 2 were laid from the input device 38 through the interface unit 4218. Alternatively, the arithmetic unit 4211 may accept information indicating a time when poultry raised in a production site are light up and a time when eggs 2 are carried in, and may calculate an estimation value of the elapsed time from the egg-laying based on the accepted information. The processing at S222 corresponds to a second information acquisition unit.

Depending on the elapsed time from the egg-laying, the state of the eggs 2 varies. For example, as the elapsed time is longer, a stain on an egg caused by washing residual upon washing the egg is more liable to remain, resulting in a higher probability of presence of dirt. Thus, images obtained by photographing eggs 2 are different depending on the elapsed time from the egg-laying. In order to accurately determine the state of the eggs 2 and sort the eggs 2, it is desirable to adjust the learning model 4215 used for determining the state of the egg 2 according to the elapsed time from when the egg 2 is laid. Alternatively, adjustment of the criteria for deciding the classification of an egg 2 depending on the determined state of the egg 2 also enables suitable sorting of the eggs 2.

The arithmetic unit 4211 next selects a learning model to be used in the processing at S232 (S223). The learning model 4215 includes multiple learning models different in parameters. At S223, the arithmetic unit 4211 selects a learning model to be used in the processing at S232 from the multiple learning models included in the learning model 4215. More specifically, the arithmetic unit 4211 selects a suitable learning model depending on the quality of eggs 2 to be shipped and the elapsed time from the egg-laying. In the processing at S232, the learning model selected in the processing at S223 is used to thereby suitably determine the state of the eggs 2.

The arithmetic unit 4211 then adjusts criteria for sorting eggs 2 to be used in processing at S25 (S224). At S224, the arithmetic unit 4211 adjusts the criteria for sorting eggs 2 depending on the quality of eggs 2 to be shipped and on the elapsed time from the egg-laying. For example, the arithmetic unit 4211 changes the criterion for dirt on the eggshell to be used for deciding the classification of an egg 2. In the processing at S25, the criterion adjusted at S224 is used to thereby suitably decide the classification of the egg 2. The processing at S223 and S224 respectively correspond to a first adjustment unit and a second adjustment unit. After completion of S224, the arithmetic unit 4211 returns the processing to the main processing.

Note that the arithmetic unit 4211 may execute either one of the processing at S223 and S224. In addition, the arithmetic unit 4211 may execute either one of the processing at S221 and S222 and execute processing at S223 or S224 based on the information acquired by the executed processing. Furthermore, the information acquired at processing at S221 and S222 are information common to multiple eggs 2, so that the arithmetic unit 4211 may perform the processing at S22 aside from the processing at S21 and S23 to S26 to be performed on each of the eggs 2. For example, before conveying multiple eggs 2, the analysis unit 421 performs the processing at S22.

Moreover, the egg image analysis device 42 may measure the shape or the size of each of the eggs 2. The arithmetic unit 4211 performs image analysis on image data to measure lengths of a longitudinal axis and a lateral axis of the egg 2 as values indicating the shape of the egg 2 based on the image data. Alternatively, the arithmetic unit 4211 performs image analysis on image data to measure an area of the egg 2 as a value indicating the size of the egg 2 based on the image data. The arithmetic unit 4211 outputs information indicating the shape or the size of the measured egg 2 through the interface unit 4218. The tap analysis device 5, for example, is connected to the interface unit 4218. The tap analysis device 5 acquires the information indicating the shape or the size of the egg 2 and uses it in the processing of determining the state of the eggshell.

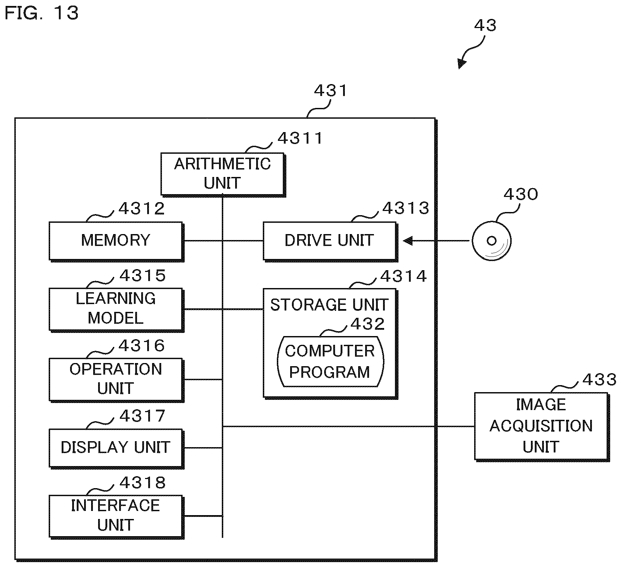

Next, the inside-package-image analysis device 43 will be described. FIG. 13 is a block diagram illustrating an example of the functional configuration of the inside-package-image analysis device 43. The inside-package-image analysis device 43 is provided with an analysis unit 431 for performing information processing and an image acquisition unit 433 for acquiring an image obtained by photographing multiple eggs 2 contained in a package. The image acquisition unit 433 is connected to the analysis unit 431.

The analysis unit 431 is configured by using a computer.
The analysis unit 431 is provided with an arithmetic unit 4311, a memory 4312, a driving unit 4313 and a nonvolatile storage unit 4314. The arithmetic unit 4311 may be configured by using a CPU, a GPU, a multicore CPU or a quantum computer, for example. The memory 4312 is a RAM, for example. Furthermore, the analysis unit 431 is provided with a learning model 4315 to be used for determining the state of eggs 2 according to image data representing an image obtained by photographing the eggs 2. Moreover, the analysis unit 431 is provided with an operation unit 4316 for accepting an operation from the user, a display unit 4317 for displaying an image and an interface unit 4318. The interface unit 4318 is connected to the travel amount detection device 36 and the input device 38. The analysis unit 431 inputs and outputs information to and from the travel amount detection device 36 and the input device 38 through the interface unit 4318.

The arithmetic unit 4311 causes the drive unit 4313 to read a computer program 432 recorded in a recording medium 430 such as an optical disk or the like and store the read computer program 432 in the storage unit 4314. The arithmetic unit 4311 executes processing according to the computer program 432 stored in the storage unit 4314. Note that the computer program 432 may be downloaded from outside of the analysis unit 431. Here, the analysis unit 431 needs not be provided with the drive unit 4313.

The learning model 4315 is a trained learning model for performing processing of determining the state of eggs 2 according to the image data. The learning model 4315 is achieved by the arithmetic unit 4311 executing information processing according to the computer program 432. Alternatively, the learning model 4315 may be configured to include a processor and a memory storing a necessary program and data. The learning model 4315 may be achieved by using a quantum computer.

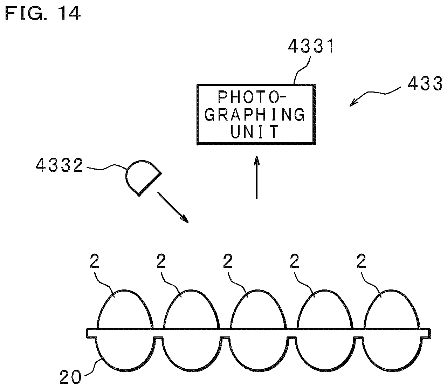

FIG. 14 is a schematic view illustrating an example of the configuration of the image acquisition unit 433. The image acquisition unit 433 includes an illumination unit 4332 illuminating multiple eggs 2 contained in a package 20 and a photographing unit 4331 photographing the multiple eggs 2 contained in the package 20. The illumination unit 4332 is configured by a light source. The illumination unit 4332 emits light and irradiates the package 20 containing the multiple eggs 2 with light. The photographing unit 4331 photographs the eggs 2 by using reflected light reflected by the eggs 2 and the package 20. In FIG. 14, light is illustrated by an arrow. The illumination unit 4332 and the photographing unit 4331 are disposed above the package 20 conveyed by the conveyor 13, for example. Note that the illumination unit 4332 may irradiate the eggs 2 with light passing through the package 20. Furthermore, the photographing unit 4331 may photograph the eggs 2 via the package 20.

The photographing unit 4331 creates image data representing an image obtained by photographing the multiple eggs 2 contained in the package 20. The photographing unit 4331 inputs the image data to the analysis unit 431. The image acquisition unit 433 corresponds to an image data acquisition unit. The image acquisition unit 433 may include an optical component (not illustrated) such as a mirror, a lens, an optical filter or the like. The image acquisition unit 433 may utilize light from outside without being provided with the illumination unit 4332. Note that the image acquisition unit 433 may be configured to acquire an image obtained by photographing the eggs 2 using transmitted light transmitted through the eggs 2 or fluorescence. Alternatively, the image acquisition unit 433 may be configured to acquire two or more images out of the image obtained by photographing the eggs 2 using reflected light, the image obtained by photographing the eggs 2 using transmitted light and the image obtained by photographing the eggs 2 using fluorescence.

The analysis unit 431 performs processing of determining the states of the eggs 2 contained in the package 20 based on the image data and of deciding a classification of the eggs 2 contained in the package 20 according to the determination result. The states of the eggs 2 to be determined specifically include the presence or absence of damage of the eggshell, the size of damage, the presence or absence of dirt on the eggshell, the degree of dirt or a type of dirt. The images obtained by photographing the eggs 2 are different depending on the states of the eggs 2. Thus, the states of the eggs 2 contained in the package 20 can be determined according to the image data.

The processing procedure for sorting eggs 2 performed by the analysis unit 431 of the inside-package-image analysis device 43 is similar to the processing procedure performed at S21 to S26 shown in the flowchart in FIG. 9. The arithmetic unit 4311 of the analysis unit 431 executes the following processing according to the computer program 432. The arithmetic unit 4311 specifies a location of the package 20 containing the eggs 2 based on the information from the travel amount detection device 36. The arithmetic unit 4311 acquires image data for any one of the packages 20 the location of which is specified from the image acquisition unit 433 (S21). The arithmetic unit 4311 stores the image data in the storage unit 4314. The arithmetic unit 4311 then performs setting processing of making setting for sorting eggs 2 (S22).

The procedure of the setting processing is similar to the procedure of the processing at S221 to S224 shown by the flowchart in FIG. 12. The arithmetic unit 4311 acquires information designating the quality of eggs 2 to be shipped (S221). For example, the arithmetic unit 4311 accepts information designating the quality set from the input device 38 through the interface unit 4318.

The arithmetic unit 4311 then selects a learning model to be used in processing at S232 (S223). The learning model 4315 includes multiple learning models different in parameters. At S223, the arithmetic unit 4311 selects a learning model to be used in the processing at S232 from the multiple learning models included in the learning model 4315. More specifically, the arithmetic unit 4311 selects a suitable learning model depending on the quality of eggs 2 to be shipped.

The arithmetic unit 4311 then adjusts the criteria for sorting eggs 2 to be used in processing at S25 (S224). At S224, the arithmetic unit 4311 adjusts the criteria for sorting eggs 2 depending on the quality of eggs 2 to be shipped. For example, the arithmetic unit 4311 changes a criterion for damage of the eggshell or a criterion for dirt on the eggshell to be utilized for deciding the classification of the eggs 2.

Note that the arithmetic unit 4311 may acquire information indicating an environment to select a learning model to be used in the processing or to adjust the criteria for sorting eggs 2. The arithmetic unit 4311 acquires package information indicating a type of the package 20 as information indicating an environment. The inside-package-image analysis device 43 includes an environmental information acquisition unit for acquiring information indicating an environment. For example, the environmental information acquisition unit is an operation unit 4316 for accepting an operation from the user. The arithmetic unit 4311 selects a learning model to be used in the determination processing out of the multiple learning models according to the information acquired by the environmental information acquisition unit in the processing at S223. Alternatively, the arithmetic unit 4311 adjusts the criteria for deciding a classification of eggs 2 depending on the determination result according to the information acquired by the environmental information acquisition unit in the processing at S224.

After completion of S224, the arithmetic unit 4311 returns the processing to the main processing. Note that the arithmetic unit 4311 may execute either one of the processing at S223 and S224. In addition, the arithmetic unit 4311 may execute a part of the processing at S221 and S222 and execute the processing at S223 or S224 based on the information acquired by the executed processing. The arithmetic unit 4311 may execute the processing at S21 and S22 in the reverse order. Furthermore, the arithmetic unit 4311 may perform the processing at S22 aside from the processing at S21 and S23 to S26 to be performed on each of the packages 20. For example, before conveying the multiple eggs 2, the arithmetic unit 4311 performs the processing at S22.

The arithmetic unit 4311 next performs determination processing for determining the state of the eggs 2 contained in the package 20 based on the image data (S23). The procedure of the determination processing is similar to the processing procedure at S231 to S233 shown by the flowchart in FIG. 10. The arithmetic unit 4311 determines the state of the eggs 2 based on a rule (S231). At step S231, the arithmetic unit 4311 determines the state of the eggs 2 contained in the package 20 according to the image data based on a predefined rule. The arithmetic unit 4311 may perform processing of determining the state of the eggs 2 by a conventionally known method. For example, the arithmetic unit 4311 determines the presence or absence of damage of the eggshells, the size of damage, the presence or absence of dirt on the eggshells, the degree of dirt or the type of dirt by the conventional image processing.

The arithmetic unit 4311 next determines the state of the eggs 2 contained in the package 20 by using the learning model 4315 (S232). The learning model 4315 is previously so trained by using training data including image data representing an image obtained by photographing the eggs 2 contained in the package 20 and the information indicating the presence or absence of damage of the eggshells, the size of damage, the presence or absence of dirt on the eggshells, the degree of dirt or the types of dirt, as to determine the presence or absence of damage of the eggshells of the eggs, the size of damage, the presence or absence of dirt on the eggshells, the degree of dirt, or the type of dirt from the image data. For example, the learning model 4315 employs a neural network.

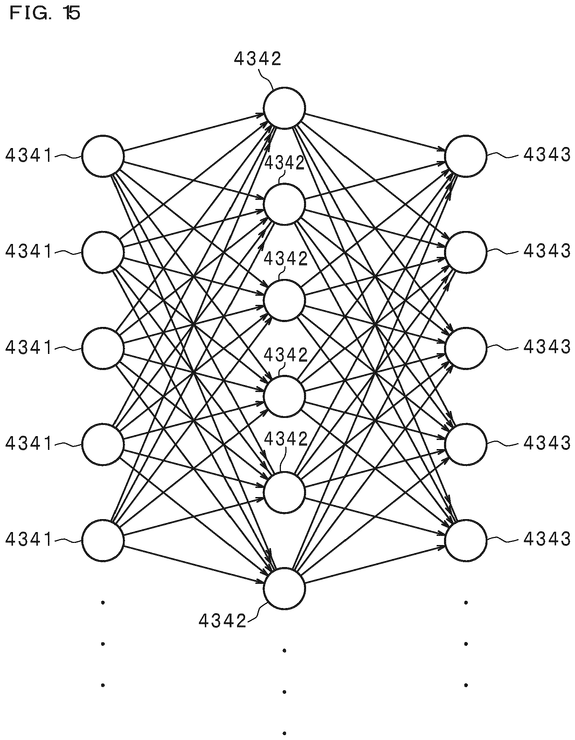

FIG. 15 is a conceptual diagram illustrating an example of the functional configuration of the learning model 4315. The learning model 4315 employs a neural network. The input layer has multiple nodes 4341 to which image data is input. For example, a pixel value of each of the pixels included in the image obtained by photographing the multiple eggs 2 contained in the package 20 is input to any of the nodes 4341. Information designating the quality may also be input to the input layer in addition to the image data. The intermediate layer has multiple nodes 4342 each performing arithmetic on the data input from each of the nodes 4341 of the input layer by using a parameter. The multiple nodes 4342 respectively output data to multiple nodes 4343 included in the output layer. The output layer has the multiple nodes 4343 each accepting data on which arithmetic has been performed from each of the nodes 4342 of the intermediate layer and outputting the state of each of the eggs 2. For example, one of the nodes 4343 outputs a probability of presence of damage on the eggshells as a score, and another one of the nodes 4343 outputs a probability of the size of damage having a certain size as a score. For example, another one of the nodes 4343 outputs a probability of presence of dirt on the eggs 2 as a score, another node outputs a probability of the degree of dirt being a predetermined degree as a score, and another one of the nodes 4343 outputs a probability of the type of dirt being a specific type as a score. The intermediate layer may include multiple layers. The learning model 4315 may employ CNN or RNN as a neural network.

The learning model 4315 is trained in advance by using the training data. As to a large number of eggs 2 the states of which have already been identified and which include eggs 2 having the eggshells with no damage and no dirt, image data of the eggs 2 contained in the package 20 are acquired to create training data. The training data includes information indicating the states of the eggs 2 contained in the package 20 and the image data representing an image obtained by photographing the eggs 2 contained in the package 20. By training, an arithmetic parameter of each of the nodes is adjusted such that when the image data as to each of the eggs 2 included in the training data is input to each of the nodes 4241 of the input layer, each of the nodes 4343 of the output layer outputs a score corresponding to the state of each of the eggs 2 indicated by the information included in the training data. For example, in the training, an error is calculated by using an error function regarding the output value from each of the nodes 4343 in the output layer and the output value predicted from the training data as variables. The arithmetic parameter of each of the nodes is adjusted such that the error is minimized by a back propagation.

The learning of the learning model 4315 may be performed by the inside-package-image analysis device 43. For example, the image acquisition unit 433 acquires the image data obtained by photographing the eggs 2 contained in the package 20 the state of which has already been identified by the image acquisition unit 433 to create training data, and the analysis unit 431 executes information processing for training. Alternatively, the inside-package-image analysis device 43 may acquire training data input from outside thereof, and the analysis unit 431 may execute information processing for training. Alternatively, training is performed by a training device located outside the inside-package-image analysis device 43, and the learning model 4315 may be created based on the training result. The training device is configured by a computer. Image data of multiple eggs 2 contained in the package 20 is acquired, the state of the eggs 2 after the image data has been acquired is checked, and training data including the image data and information indicating the checked state of the eggs 2 may be created. The training device may receive the training data and may execute training. The training device may receive the image data and the information indicating the state of the eggs 2, create training data including the input data and information, and perform training by using the created training data.

The learning model 4315 may be configured to receive an input of image data representing an image obtained by photographing eggs 2 using transmitted light or fluorescence and output the state of the eggs 2. Alternatively, the learning model 4315 may include two or more learning models out of the learning model to which image data representing an image obtained by photographing the eggs 2 using reflected light are to be input, the learning model to which image data representing an image obtained by photographing the eggs 2 using transmitted light are to be input and the learning model to which image data representing an image obtained by photographing the eggs 2 using fluorescence are to be input.

The arithmetic unit 4311 determines the state of the eggs 2 according to the output of the learning model 4315. For example, the arithmetic unit 4311 determines that the eggshells have no damage if the probability of presence of damage on the eggshells is less than a predetermined threshold and determines that the eggshells have damage if the probability of presence of damage on the eggshells is equal to or larger than the predetermined threshold. For example, the arithmetic unit 4311 determines the degree of damage having the maximum score to be the size of damage of the eggshells. The arithmetic unit 4311 determines that the eggshells have dirt if the probability of presence of dirt on the eggshells is equal to or larger than a predetermined threshold, and determines that the eggshells have no dirt if the probability of presence of dirt on the eggshells is less than the predetermined threshold, for example. For example, the arithmetic unit 4311 determines the degree of dirt having the maximum score to be the degree of dirt on the eggshells. For example, the arithmetic unit 4311 determines the type of dirt having the maximum score to be the type of the dirt on the eggshells.

Furthermore, the learning model 4315 may individually include a learning model for determining the presence or absence of damage of the eggshells, a learning model for determining the size of damage of the eggshells, a learning model for determining the presence or absence of dirt on the eggshells, a learning model for determining the degree of dirt on the eggshells, or a learning model for determining the type of dirt on the eggshells. At S232, the arithmetic unit 4311 performs each determination by using each of the learning models.

The arithmetic unit 4311 may determine the state of the eggs 2 by a combination of two or more outputs out of the output of the learning model to which image data representing an image obtained by photographing the eggs 2 using reflected light is input, the output of the learning model to which image data representing an image obtained by photographing the egg 2 using transmitted light is input, and the output of the learning model to which image data representing an image obtained by photographing the egg 2 using fluorescence is input. At step S232, the arithmetic unit 4311 uses the learning model selected at S223. The processing at step S231 and at step S232 may be executed in the reverse order or may be executed in parallel.

The arithmetic unit 4311 then decides the determination result of the state of the eggs 2 contained in the package 20 based on the determination result obtained at S231 and the determination result obtained at S232 (S233). At S233, the determination result of the eggs 2 is decided by a combination of the rule-based determination result obtained at S231 and the determination result using the learning model 4315 obtained at S232. The method of combining the rule-based determination result and the determination result using the learning model is similar to the method in the processing at S123. After completion of S233, the arithmetic unit 4311 returns the processing to the main processing. The combined use of the rule-based determination and the determination using the learning model 4315 enables suitable determination.

The arithmetic unit 4311 next outputs the determination result of the state of the eggs 2 contained in the package 20 (S24). At step S24, the arithmetic unit 4311 displays an image including the determination result on the display unit 4317. For example, the arithmetic unit 4311 displays the number of eggs 2 with the eggshells having damage or the number of eggs 2 with the eggshells having dirt on the display unit 4317. The arithmetic unit 4311 may display the rule-based determination result obtained at step S231 and the determination result using the learning model 4315 obtained at S122 on the display unit 4317.

The arithmetic unit 4311 then decides a classification of the eggs 2 contained in the package 20 depending on the determination result of the state of the eggs 2 contained in the package 20 (S25). The arithmetic unit 4311 decides the classification of the eggs 2 by a unit of the package 20. For example, if there is an egg 2 with the eggshell having the degree of damage being above a predetermined criterion out of the multiple eggs 2 contained in the package 20 or if there is an egg 2 with the eggshell having the degree of dirt being above a predetermined criterion out of the multiple eggs 2 contained in the package 20, the arithmetic unit 4311 specifies this package and moves it to a place different from that for the packages 20 to be shipped to general stores. For example, if all the eggs contained in the package 20 have neither damage nor dirt on the eggshells or if the degree of damage or the degree of dirt of the eggshell of all the eggs 2 contained in the package 20 is equal to or below a predetermined criterion, the arithmetic unit 4311 decides the classification of the multiple eggs 2 contained in the package 20 are categorized as a group for the eggs 2 allowed to be shipped to a general store. Furthermore, the arithmetic unit 4311 may sort the eggs 2 depending on the type of dirt on the eggshells. At S25, the arithmetic unit 4311 executes the processing by using the criteria for sorting eggs 2 adjusted at S224.

The arithmetic unit 4311 next outputs classification data indicating a classification of the eggs 2 contained in the package 20 through the interface unit 4318 (S26). For example, the arithmetic unit 4311 displays an image representing the classification of the eggs 2 on the display unit 4317. If an image representing that an egg 2 contained in the package 20 is categorized as an egg 2 unsuitable for the egg 2 to be shipped to the general stores is displayed, the user picks the egg 2 out from the package 20. Note that the egg sorting system 100 may be provided with a device (not illustrated) for separating the package 20 containing an egg 2 unsuitable for the egg 2 to be shipped to the general stores. The device separates the package 20 based on the classification data output from the inside-package-image analysis device 43. The analysis unit 431 executes the processing at S21 to S26 for each of the packages 20 conveyed by the conveyor 13. The analysis unit 431 may perform the processing for the multiple packages 20 successively or in parallel.

As described in detail above, in the present embodiment, the broken egg detection device 41, the egg image analysis device 42 or the inside-package-image analysis device 43 acquires image data representing an image obtained by photographing an egg 2, determines the state of the egg 2 by using a learning model depending on the image data and decides a classification of the egg 2 according to the determination result. The use of the learning model for determining the state of each of the eggs 2 enables more accurate determination of the state of the egg 2 and more suitable sorting of the eggs 2 even if various eggs 2 in different states coexist. In the present embodiment, the broken egg detection device 41, the egg image analysis device 42 or the inside-package-image analysis device 43 determines as the states of eggs 2 the presence or absence of damage on the eggshell, the degree of damage of the eggshell, a color of the eggshell, evenness of the color, the presence or absence of dirt on the eggshell, the degree of dirt, the type of dirt, the presence or absence of a deformity of the eggshell, the type of the deformity, surface properties of the eggshell, a pattern of the eggshell or a printing on the eggshell. These states of the eggs 2 have much effect on the quality of the eggs 2. By sorting the eggs 2 depending on the states of the eggs 2, eggs 2 with a suitable quality can be selected.

In addition, the inside-package-image analysis device 43 determines the state of eggs 2 according to image data representing an image obtained by photographing the eggs 2 contained in the package 20 and sorts the eggs 2. For the eggs 2 contained in the package 20 just before shipping, if an egg 2 with the damaged eggshell or an egg 2 with dirt on the eggshell is included in the multiple eggs 2 contained in the package, the product quality consisting of the multiple eggs 2 is degraded. By determining the degree of damage of the eggshell, the degree of dirt on the eggshell or the type of the dirt on the eggshell with the eggs 2 contained in the package 20 to sort the egg 2, it is possible to prevent the eggs 2 of poor quality from being shipped.

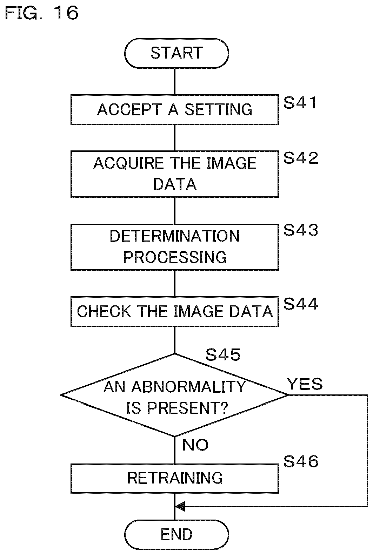

In the present embodiment, the egg image analysis device 42 can retrain the learning model 4215. The analysis unit 421 of the egg image analysis device 42 executes processing for retraining. FIG. 16 is a flowchart showing a processing procedure for retraining. The arithmetic unit 4211 of the analysis unit 421 executes the following processing according to the computer program 422. Upon retraining, image data representing an image obtained by photographing an egg 2 with an identified state is obtained, and retraining is performed using information indicating the state of the egg and the acquired image data as training data. First, the user places an egg 2 with an identified state on the conveyance path of the conveyance device 11 while the operation of the conveyance device 11 is suspended. The analysis unit 421 accepts a setting related to the egg 2 with an identified state including the information indicating the state of the egg 2 by the user operating the operation unit 4216 (S41). The arithmetic unit 4211 stores the data indicating the accepted setting in the storage unit 4214. The processing at S41 corresponds to an acceptance unit.

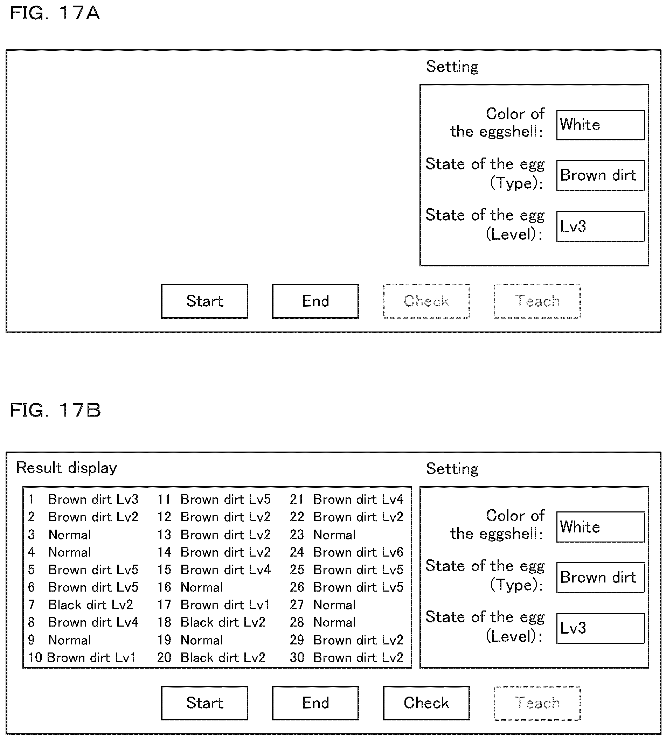

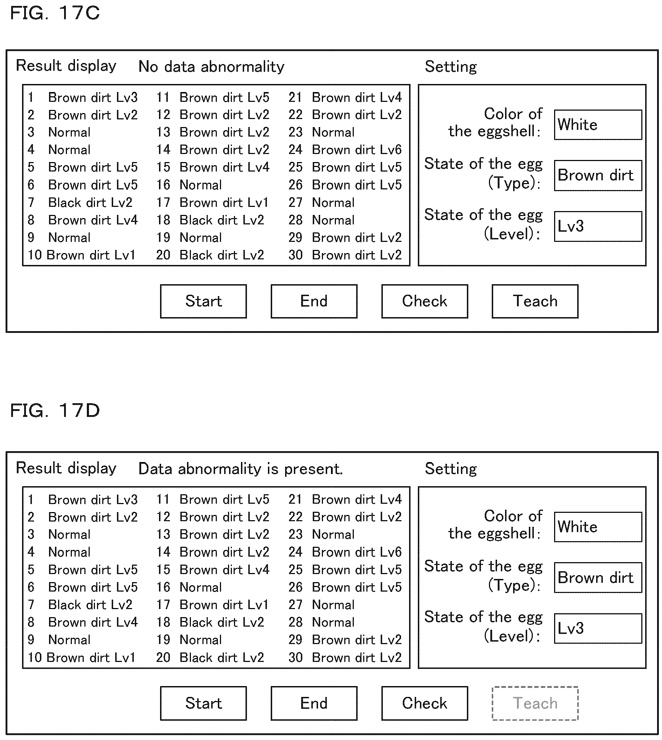

FIGs. 17A, 17B, 17C and 17D are schematic views illustrating screen examples to be displayed on the display 4217 upon retraining. FIGs. 17A, 17B, 17C and 17D each illustrate an example of a touch panel composed of the operation unit 4216 and the display unit 4217, each including operation buttons as a part of the operation unit 4216 on the screen. FIG. 17A is a screen example to be displayed on the display unit 4217 when a setting is made. The setting is input by the user operating the operation unit 4216. The information indicating the state of the egg 2 disposed is input. FIGs. 17A, 17B, 17C and 17D each illustrate an example in which inputted is that the color of the eggshell of the egg 2 is white, the type of the state of the egg 2 is brown dirt, and the level of the state of the egg 2 is Lv3. The Lv means a level. The numerical value of the level means a degree of dirt on the eggshell. The greater the numerical value of the level is, the heavier the degree of dirt on the eggshell is. In the example in FIG. 17A, input is a setting in which the color of the eggshell of the egg 2 is white, the type of the dirt on the eggshell is brown dirt, and the degree of dirt is Lv3.

The arithmetic unit 4211 next acquires the image data (S42). At step S42, the arithmetic unit 4211 accepts an instruction of acquiring the image data by the user operating the operation unit 4216, such as pressing a start button by the user on the screen shown in FIG. 17. The conveyance device 11 conveys eggs 2, and the arithmetic unit 4211 acquires image data representing an image obtained by photographing the eggs 2 from the image acquisition unit 423 similarly to the processing at S21. The arithmetic unit 4211 stores the image data in the storage unit 4214.

The arithmetic unit 4211 then performs determination processing (S43). At S43, the arithmetic unit 4211 accepts an instruction of determination by the user operating the operation unit 4216, such as pressing an end button by the user on the screen illustrated in FIG. 17A. The arithmetic unit 4211 performs determination processing of determining the state of the eggs 2 based on the acquired image data similarly to the processing at S23. The arithmetic unit 4211 displays the determination result of the states of the eggs 2 on the display unit 4217. FIG. 17B illustrates a screen example in which the determination result is displayed. For each of the eggs 2, the determination result of the state of each of the eggs 2 is shown. The normal egg means that there is no dirt on the eggshell. In the example in FIG. 17B, though the set degree of dirt on the eggshell is Lv3, many eggs 2 having the determination results different from Lv3 are shown, which suggests inaccurate determination.

The arithmetic unit 4211 then checks the image data (S44). In some case, image data is abnormal due to a malfunction of equipment such as the image acquisition unit 423, stray light or the like. If the image data is abnormal, retraining using the image data results in inaccurate training. Thus, it is required to check the image data. At S44, the arithmetic unit 421 accepts an instruction of checking by the user operating the operation unit 4216, such as pressing a check button by the user on the screen illustrated in FIG. 17B. The arithmetic unit 4211 checks the image data by a method of determining the presence or absence of malfunction of equipment or a method of determining the presence or absence of an abnormality in the image data based on a predetermined criterion.

The arithmetic unit 4211 then determines whether or not an abnormality is present in the image data as a result of checking (S45). If an abnormality is present (S45: YES), the arithmetic unit 4211 displays the presence of the abnormality on the display unit 4217 and ends the processing. If an abnormality is absent (S45: NO), the arithmetic unit 4211 displays the absence of an abnormality on the display unit 4217 and then performs retraining processing (S46).

FIG. 17C illustrates a screen example in which the image data includes no abnormality. The absence of an abnormality in the image data is displayed, and a teach button is available. The arithmetic unit 421 accepts an instruction of retraining by the user operating the operation unit 4216, such as pressing the teach button by the user on the screen illustrated in FIG. 17C. FIG. 17D illustrates a screen example in which the image data includes an abnormality. The presence of an abnormality in the image data is displayed. The teach button is made unavailable, which makes it impossible for the user to input an instruction of retraining.

At S46, the arithmetic unit 4211 performs machine learning of the learning model 4215 using a relation between the information indicating the state of the eggs 2 included in the setting accepted at S41 and the image data for each of the eggs 2 obtained at S42 as training data. The learning model 4215 performs machine learning for the parameter of each of the nodes 4242 in the intermediate layer based on the training data such that when the image data is input to the nodes 4241 of the input layer, the state of the egg 2 according to the setting can be output from the nodes 4243 of the output layer. By the machine learning, the retrained learning model 4215 can be obtained. The processing at S46 corresponds to a retraining processing unit.

The analysis unit 421 then ends the retraining processing. The processing at S41 to S46 is executed as necessary. In the processing at S41 to S46, if an abnormality is present in any of the image data for multiple eggs 2, retraining of the learning model 4215 is not performed. Alternatively, if eggs 2 of the image data containing an abnormality and eggs 2 of the image data containing no abnormality coexist, the analysis unit 421 may perform retraining on only the eggs 2 of the image data containing no abnormality.

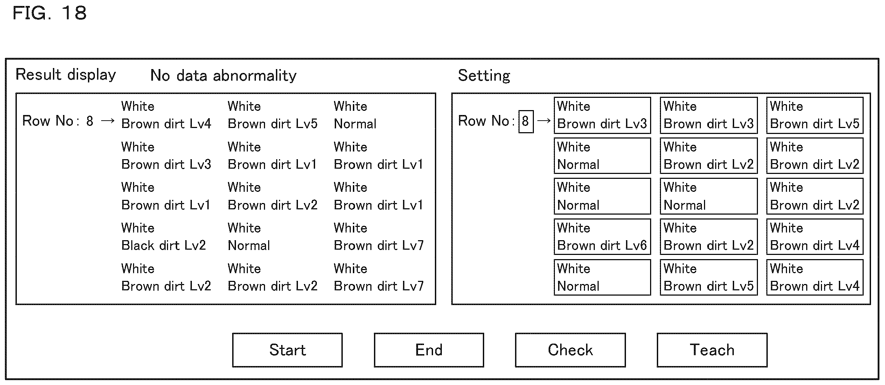

FIGs. 17A, 17B, 17C and 17D each show an example in which the egg image analysis device 42 performs retraining by using multiple eggs 2 categorized in the same state, but the egg image analysis device 42 may be configured to perform retraining using multiple eggs categorized in different states. FIG. 18 is a schematic view illustrating another screen example to be displayed on the display unit 4217 when retraining is performed. For each of the eggs 2 aligned in multiple rows, the color of the eggshell, the type of dirt, the degree of dirt are input. In order to indicate the location of the eggs 2, a row number (No.) forming one of the multiple rows is input. Similarly to the examples in FIGs. 17A, 17B, 17C and 17D, image data is acquired, determination is performed, and the image data is checked. And retraining is performed using information indicating the state of each of the eggs 2 and the image data of an image obtained by photographing each of the eggs 2 as training data.

Though FIGs. 17A, 17B, 17C, 17D and FIG. 18 each illustrate an example in which retraining related to the color and dirt on the eggshells is performed, the egg image analysis device 42 may perform retraining related to the presence or absence of damage of the eggshells, the degree of damage, the evenness of a color of the eggshells, the presence or absence of a deformity of the eggshells, the type of a deformity, surface properties of the eggshells, a pattern of the eggshells or a printing on the eggshells. In the retraining processing, the egg image analysis device 42 first acquires image data representing an image of eggs 2 and may then make a setting as to the state of an egg 2 thereafter. Alternatively, the egg image analysis device 42 may modify the state of the egg 2 originally set into a different state and then perform retraining.

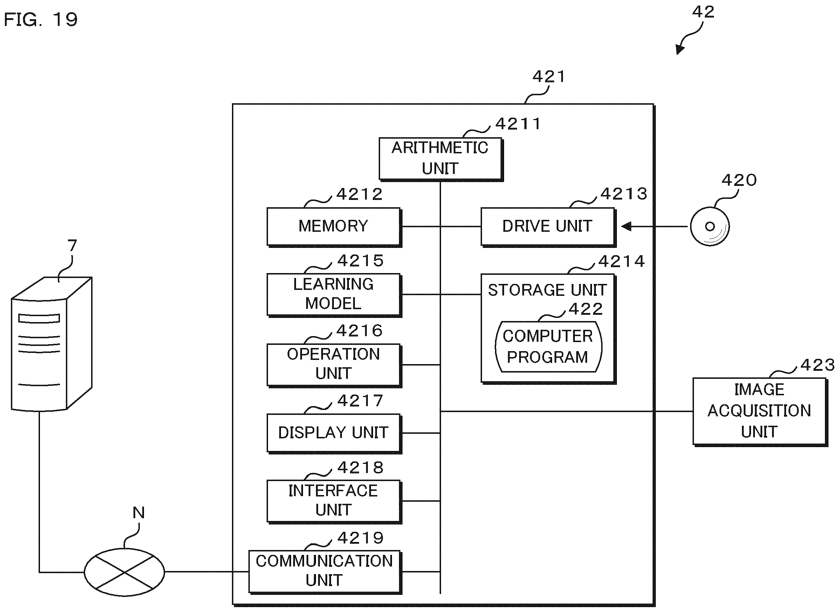

Note that the egg image analysis device 42 may be configured to perform training outside thereof. FIG. 19 is a block diagram illustrating an example of the functional configuration of the egg image analysis device 42 which training is performed outside thereof. The analysis unit 421 is provided with a communication unit 4219. The communication unit 4219 is connected to a communication network N such as the Internet or the like. The communication network N is connected to the storage device 7. The analysis unit 421 makes communication with the storage device 7 via the communication network N by the communication unit 4219.

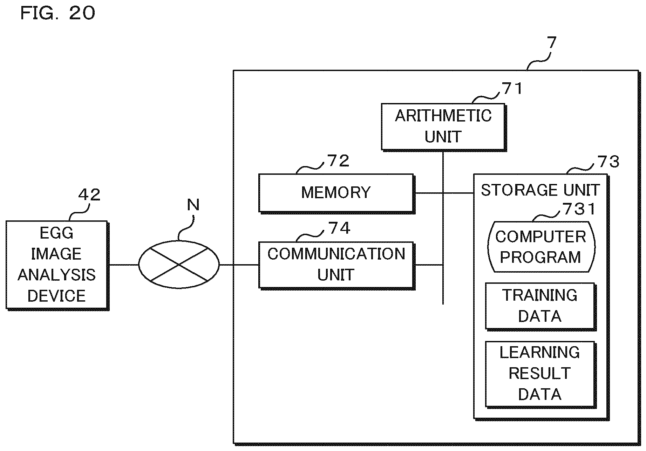

FIG. 20 is a block diagram illustrating an example of the internal configuration of the storage device 7. The storage device 7 is a computer such as a server device or the like. The storage device 7 is provided with an arithmetic unit 71, a memory 72, a nonvolatile storage unit 73 such as a hard disk or the like and a communication unit 74. The communication unit 74 is connected to the communication network N. The storage unit 73 stores a computer program 731. The arithmetic unit 71 executes processing according to the computer program 731.

At S46, the analysis unit 421 transmits to the storage device 7 the training data including the information indicating the state of the egg 2 included in the setting accepted at S41 and the image data of each of the eggs 2. The storage device 7 receives the training data through the communication unit 74 and stores the training data in the storage unit 73. The arithmetic unit 71 performs machine learning of the learning model by using the training data and stores learning result data indicating the learning result of the learning model in the storage unit 73. Furthermore, the arithmetic unit 71 causes the communication unit 74 to transmit the learning result data to the egg image analysis device 42. The analysis unit 421 receives the learning result data through a communication unit 4219, and the arithmetic unit 211 updates the learning model 4215 based on the learning result data. Hence, retraining of the learning model 4215 is thus performed.

The broken egg detection device 41 and the inside-package-image analysis device 43 can also retrain the learning model by executing the processing at S41 to S46 as in the egg image analysis device 42. The broken egg detection device retrains the learning model 4115. The inside-package-image analysis device 43 retrains the learning model 4315.

As described above in detail, in the present embodiment, the broken egg detection device 41, the egg image analysis device 42 and the inside-package-image analysis device 43 each receive information indicating the state of each of the eggs 2, acquire image data representing an image obtained by photographing the egg 2 and retrain the learning model by using the information indicating the state of the egg 2 and the image data as training data. The learning model is retrained according to the state of actual eggs 2, so that the learning model is updated so as to accurately determine the state of the eggs 2. Thus, it becomes possible to more accurately determine the state of the eggs 2 and more suitably sort the eggs 2.

In the present embodiment, the broken egg detection device 41, the egg image analysis device 42 and the inside-package-image analysis device 43 are configured to determine the state of each of the eggs 2 by themselves. The egg sorting system 100 may be configured to determine the state of each of the eggs 2 outside the broken egg detection device 41, the egg image analysis device 42 and the inside-package-image analysis device 43. For example, a determination device including a trained learning model is provided outside the broken egg detection device 41, the egg image analysis device 42 or the inside-package-image analysis device 43. The broken egg detection device 41, the egg image analysis device 42 or the inside-package-image analysis device 43 acquires image data and inputs the image data to the determination device. The determination device determines the state each of the eggs 2 according to the input image data by the learning model. The determination device inputs the determination result to the broken egg detection device 41, the egg image analysis device 42 or the inside-package-image analysis device 43, and the broken egg detection device 41, the egg image analysis device 42 or the inside-package-image analysis device 43 decides a classification of each of the eggs 2 according to the input determination result. The determination device is configured by a computer. The determination device may be achieved by using multiple computers or using a cloud.

It is to be understood that the embodiments disclosed here are illustrative in all respects and not restrictive. The scope of the present invention is defined by the appended claims, not by the above-mentioned meaning, and all changes that fall within the meanings and the bounds of the claims, or equivalence of such meanings and bounds are intended to be embraced by the claims.

### (Note 1)

An egg sorting system for sorting eggs, comprises:
a first egg sorting device and a second egg sorting device that decide a classification of eggs; and
a distribution device that distributes eggs according to a classification decided by the first egg sorting device or a classification decided by the second egg sorting device, wherein
the first egg sorting device includes:
   an image data acquisition unit that acquires image data representing an image obtained by photographing an egg;
   a determination unit that determines a state of the egg according to the image data acquired by the image data acquisition unit by a learning model trained using training data including information indicating a state of an egg and image data related to the egg; and
   a decision unit that decides a classification of an egg according to a determination result of a state of the egg,
the second egg sorting device includes a learning model trained by using training data including tapping data indicating a phenomenon caused by tapping an egg and information indicating the state of an eggshell, and
the second egg sorting device acquires tapping data indicating a phenomenon caused by tapping an egg, determines the state of an eggshell based on the tapping data acquired using the learning model, and decides a classification of the egg based on the determination result of the state of the eggshell.

### (Note 2)

The egg sorting system according to Note 1, further comprises a third egg sorting device that decides a classification of eggs, wherein
the third egg sorting device includes a deep learning model trained by training data including information indicating an interior state of an egg and transmitted light data indicating light transmitted through the egg,
the third egg sorting device acquires transmitted light data indicating light transmitted through an egg, determines the interior state of the egg based on the transmitted light data acquired using the deep learning model, and decides a classification of the egg depending on the determination result of the interior state of the egg, and
the distribution device distributes eggs according to a classification decided by the first egg sorting device, the second egg sorting device or the third egg sorting device.

### (Note 3)

An egg sorting system for sorting eggs, comprises:
a first egg sorting device and a third egg sorting device that decide a classification of eggs; and
a distribution device that distributes eggs according to a classification decided by the first egg sorting device or a classification decided by the third egg sorting device, wherein
the first egg sorting device includes:
   an image data acquisition unit that acquires image data representing an image obtained by photographing an egg;
   a determination unit that determines a state of the egg according to the image data acquired by the image data acquisition unit by a learning model trained using training data including information indicating a state of an egg and image data related to the egg; and
   a decision unit that decides a classification of an egg according to a determination result of a state of the egg,
the third egg sorting device includes a deep learning model trained by using training data including information indicating an interior state of an egg and transmitted light data indicating light transmitted through the egg, and
the third egg sorting device acquires transmitted light data indicating light transmitted through an egg, determines the interior state of the egg based on the transmitted light data acquired using the deep learning model, and decides a classification of the egg depending on the determination result of the interior sate of the egg.

### [Description of Reference Numerals]

11 conveyance device
12 distribution device
13, 14 conveyor
2 egg
20 package
41 broken egg detection device
42 egg image analysis device
43 inside-package-image analysis device
411, 421, 431 analysis unit
4115, 4215, 4315 learning model
412, 422, 432 computer program
413, 423, 433 image acquisition unit
4131, 4231, 4332 photographing unit
4132, 4232, 4332, illumination unit
7 storage device
N communication network

## Claims

1. An egg sorting device, comprising:
an image data acquisition unit that acquires image data representing an image obtained by photographing an egg;
a first determination unit that determines a state of the egg according to the image data acquired by the image data acquisition unit by a learning model trained using training data including information indicating a state of an egg and image data related to the egg; and
a decision unit that decides a classification of an egg according to a determination result of a state of the egg.

2. The egg sorting device according to claim 1, wherein the determination result indicates a degree of damage of an eggshell, a degree of dirt on an eggshell or a type of dirt on an eggshell.

3. The egg sorting device according to claim 1, wherein
the image data acquisition unit acquires image data representing an image obtained by photographing the egg contained in a package, and
the determination result indicates a degree of damage of an eggshell, a degree of dirt on an eggshell or a type of dirt on an eggshell.

4. The egg sorting device according to any one of claims 1 to 3, further comprising
an output unit that outputs information indicating the state of the egg determined by the first determination unit.

5. The egg sorting device according to any one of claims 1 to 4, further comprising
a second determination unit that determines a state of the egg according to the image data acquired by the image data acquisition unit based on a predefined rule, wherein
the decision unit decides a classification of the egg depending on a determination result obtained by the first determination unit and a determination result obtained by the second determination unit.

6. The egg sorting device according to any one of claims 1 to 5, further comprising:
an acceptance unit that accepts information indicating a state of an egg whose state is identified; and
a retraining unit that retrains the learning model using training data including the image data acquired by the image data acquisition unit for the same egg and the information accepted by the acceptance unit.

7. The egg sorting device according to any one of claims 1 to 6, further comprising:
a first information acquisition unit that acquires information designating quality of an egg; and
a first adjustment unit that selects a learning model to be used in the first determination unit out of multiple learning models according to the information acquired or that adjusts a criterion for the decision unit deciding a classification of an egg depending on the determination result according to the information acquired.

8. The egg sorting device according to any one of claims 1 to 7, further comprising:
a second information acquisition unit that acquires information indicating an estimated elapsed time from egg-laying; and
a second adjustment unit that selects a learning model to be used by the first determination unit out of multiple learning models according to the information acquired or that adjusts a criterion for the decision unit deciding a classification of an egg depending on the determination result according to the information acquired.

9. An egg sorting method, comprising:
acquiring image data representing an image obtained by photographing an egg;
determining a state of the egg according to the image data acquired by a learning model trained using training data including information indicating a state of an egg and image data related to the egg; and
deciding a classification of an egg according to a determination result of a state of the egg.

10. A computer program that causes a computer to execute processing of sorting eggs, causing the computer to execute a process including:
a step of determining a state of an egg according to acquired image data of the egg by a learning model trained using training data including image data representing an image obtained by photographing an egg and information indicating a state of the egg; and
a step of deciding a classification of an egg according to a determination result of a state of the egg.
